# EUROPEAN PATENT APPLICATION

(11) **EP 4 501 202 A1**
(43) Date of publication of application: **05.02.2025**
(21) Application number: 22933554.2
(22) Date of filing: 25.03.2022
(51) Int. Cl.: A61B 1/00

(54) **IMAGING DEVICE, PROGRAM, AND METHOD**

(71) Applicant: Aillis Inc., Tokyo, 1040028 (JP)
(72) Inventor: YANG, Jianxing, Tokyo 104-0028 (JP); FUKUDA, Atsushi, Tokyo 104-0028 (JP); SAKUMA, Akiho, Tokyo 104-0028 (JP)
(74) Representative: Reddie & Grose LLP
(86) International application number: PCT/JP2022/014695
(87) International publication number: WO 2023/181417

(57) **Abstract**

To provide an imaging device more suitable for capturing an image of a subject that includes at least a portion of a natural opening of a subject person.

Provided is an imaging device in which at least one processor is configured to perform a process of receiving subject person information including first subject person information associated with one or each of a plurality of subject persons including a first subject person via a communication interface from an external device communicably connected to the imaging device via a network, and in a case of outputting, as a list, pieces of subject person information of unimaged subject persons among the one or the plurality of subject persons, outputting the list without including the first subject person information before the first subject person information associated with the first subject person is registered in the external device, and outputting the list including the first subject person information after the first subject person information is registered in the external device.

## Description

### Technical Field

The present disclosure relates to an imaging device including a camera configured to capture an image of a subject, a program, and a method.

### Background Art

Conventionally, it has been known that a doctor diagnoses, for example, a viral cold or the like by observing a change in a state of an oral cavity of a subject person. Non Patent Literature 1 reports that there is a specific pattern for influenza in lymphatic follicles appearing at a deepest part of a pharynx located inside an oral cavity. The lymphatic follicles having this specific pattern are called influenza follicles, which are a characteristic sign of influenza, and are said to appear about 2 hours after onset. Therefore, it is very important to acquire an image obtained by imaging a state of the oral cavity of the subject person.

### Citation List

### Non Patent Literature

Non Patent Literature 1: Miyamoto and Watanabe, "Posterior Pharyngeal Wall Follicles as a Diagnostic Marker of Influenza During Physical Examination: Considering Their Meaning and Value" Journals of Nihon University Medical Association 72(1):11-18 (2013)

### Summary of Invention

### Technical Problem

Therefore, based on the technology as described above, an object of the present disclosure is to provide an imaging device, a program, and a method more suitable for capturing an image of a subject including at least a part of a natural opening of a subject person according to various embodiments.

### Solution to Problem

According to one aspect of the present disclosure, provided is "an imaging device including: a camera configured to capture an image of a subject including at least a part of a natural opening of one or a plurality of subject persons including a first subject person; and at least one processor, in which the at least one processor is configured to perform a process of receiving subject person information including first subject person information associated with the one or each of the plurality of subject persons including the first subject person via a communication interface from an external device communicably connected to the imaging device via a network, and in a case of outputting, as a list, pieces of subject person information of unimaged subject persons among the one or the plurality of subject persons, outputting the list without including the first subject person information before the first subject person information associated with the first subject person is registered in the external device, and outputting the list including the first subject person information after the first subject person information is registered in the external device".

According to one aspect of the present disclosure, provided is "a program that causes an imaging device including a camera configured to capture an image of a subject including at least a part of a natural opening of one or a plurality of subject persons including a first subject person to function as a processor, in which the processor is to receive subject person information including first subject person information associated with the one or each of the plurality of subject persons including the first subject person via a communication interface from an external device communicably connected to the imaging device via a network, and in a case of outputting, as a list, pieces of subject person information of unimaged subject persons among the one or the plurality of subject persons, output the list without including the first subject person information before the first subject person information associated with the first subject person is registered in the external device, and output the list including the first subject person information after the first subject person information is registered in the external device".

According to one aspect of the present disclosure, provided is "a method to be executed by at least one processor in an imaging device including: a camera configured to capture an image of a subject including at least a part of a natural opening of one or a plurality of subject persons including a first subject person; and the at least one processor, in which the method includes: a stage of receiving subject person information including first subject person information associated with the one or each of the plurality of subject persons including the first subject person via a communication interface from an external device communicably connected to the imaging device via a network; and a stage of, in a case of outputting, as a list, pieces of subject person information of unimaged subject persons among the one or the plurality of subject persons, outputting the list without including the first subject person information before the first subject person information associated with the first subject person is registered in the external device, and outputting the list including the first subject person information after the first subject person information is registered in the external device".

### Advantageous Effects of Invention

According to the present disclosure, it is possible to provide the imaging device, the program, and the method more suitable for capturing the image of the subject including at least the part of the natural opening of the subject person.

Note that the above effects are merely exemplary for convenience of description, and are not restrictive. In addition to or instead of the above effects, any effect described in the present disclosure or any effect obvious to a person skilled in the art can also be exhibited.

### Brief Description of Drawings

Fig. 1 is a view illustrating a use state of an imaging device 200 according to one embodiment of the present disclosure.
Fig. 2 is a view illustrating a use state of the imaging device 200 according to one embodiment of the present disclosure.
Fig. 3 is a block diagram illustrating a configuration of a processing system 1 according to one embodiment of the present disclosure.
Fig. 4 is a block diagram illustrating configurations of a processing device 100 and the imaging device 200 according to one embodiment of the present disclosure.
Fig. 5 is a block diagram illustrating a configuration of a server device 300 according to one embodiment of the present disclosure.
Fig. 6A is a view conceptually illustrating a subject person management table stored in the server device 300 according to one embodiment of the present disclosure.
Fig. 6B is a view conceptually illustrating an image management table stored in the server device 300 according to one embodiment of the present disclosure.
Fig. 7 is a view illustrating processing sequences to be executed among the processing device 100, the imaging device 200, and the server device 300 according to one embodiment of the present disclosure.
Fig. 8A is a chart illustrating a processing flow to be executed in the processing device 100 according to one embodiment of the present disclosure.
Fig. 8B is a chart illustrating a processing flow to be executed in the processing device 100 according to one embodiment of the present disclosure.
Fig. 8C is a chart illustrating a processing flow to be executed in the processing device 100 according to one embodiment of the present disclosure.
Fig. 9A is a chart illustrating a processing flow to be executed in the server device 300 according to one embodiment of the present disclosure.
Fig. 9B is a chart illustrating a processing flow to be executed in the server device 300 according to one embodiment of the present disclosure.
Fig. 9C is a chart illustrating a processing flow related to generation of a learned model according to one embodiment of the present disclosure.
Fig. 9D is a chart illustrating a processing flow related to the generation of the learned model according to one embodiment of the present disclosure.
Fig. 10 is a chart illustrating a processing flow to be executed in the imaging device 200 according to one embodiment of the present disclosure.
Fig. 11A is a view illustrating an example of a screen to be displayed on the processing device 100 according to one embodiment of the present disclosure.
Fig. 11B is a view illustrating an example of a screen to be displayed on the processing device 100 according to one embodiment of the present disclosure.
Fig. 11C is a view illustrating an example of a screen to be displayed on the processing device 100 according to one embodiment of the present disclosure.
Fig. 12A is a view illustrating an example of a screen to be displayed on the imaging device 200 according to one embodiment of the present disclosure.
Fig. 12B is a view illustrating an example of a screen to be displayed on the imaging device 200 according to one embodiment of the present disclosure.
Fig. 12C is a view illustrating an example of a screen to be displayed on the imaging device 200 according to one embodiment of the present disclosure.
Fig. 12D is a view illustrating an example of a screen to be displayed on the imaging device 200 according to one embodiment of the present disclosure.
Fig. 12E is a view illustrating an example of a screen to be displayed on the imaging device 200 according to one embodiment of the present disclosure.
Fig. 12F is a view illustrating an example of a screen to be displayed on the imaging device 200 according to one embodiment of the present disclosure.

### Description of Embodiments

### First Embodiment

### 1. Overview of Processing System 1

The processing system 1 according to the present disclosure is mainly used to obtain a subject image by imaging an inner portion of an oral cavity of a subject person. In particular, the processing system 1 is used to image a back of a throat area of the oral cavity, specifically, a pharynx. Accordingly, in the following description, a case where the processing system 1 according to the present disclosure is used for imaging the pharynx will be mainly described. However, the pharynx is merely an example of an imaging site, and as a matter of course, the processing system 1 according to the present disclosure can be suitably used even in other sites in the oral cavity such as tonsils and a larynx, or other natural openings such as an external auditory canal, a vagina, a rectum, and a nasal cavity.

As an example, the processing system 1 according to the present disclosure is used to determine a possibility of contracting a predetermined disease from a subject image obtained by imaging a subject including at least a pharyngeal area of the oral cavity of the subject person, and to diagnose or assist the diagnosis for the predetermined disease. An example of the disease determined by the processing system 1 is influenza. Usually, the possibility of contracting the influenza is diagnosed by examining the pharynx or a tonsil area of the subject person or determining presence or absence of findings such as follicles in the pharyngeal area. However, it is possible to perform the diagnosis or the assistance by determining the possibility of contracting the influenza using the processing system 1 and outputting a result of the determination. Note that the determination of the possibility of contracting the influenza is an example. The processing system 1 can be suitably used to determine any disease in which differences appear in findings in the natural opening due to the contracting. Note that the differences in the findings are not limited to those found by a doctor or the like, and medically known to exist. For example, a difference that can be recognized by a person other than a doctor or a difference that can be detected by artificial intelligence or image recognition technology can be suitably applied to the processing system 1.

In addition to the influenza, examples of such disease include, as a disease that is determined based on an image of a natural opening, mainly the oral cavity, the pharynx, the larynx, or the like, a hemolytic streptococcal infection, an adenovirus infection, an EB virus infection, a mycoplasma infection, infections such as a hand, foot and mouth disease, herpangina and candidiasis, diseases exhibiting vascular disorders or mucosal disorders such as arteriosclerosis, diabetes and hypertension, and tumors such as oral cancer, tongue cancer and pharyngeal cancer. Further, examples of a disease determined from an image of the external auditory canal among the natural openings include tumors such as cancer of an external auditory canal and cancer of an auditory organ, inflammation such as middle otitis and myringitis, eardrum diseases such as perforation of tympanum, and trauma. Further, examples of a disease determined from an image of the nasal cavity among the natural openings include inflammation such as rhinitis, infectious diseases such as sinusitis, tumors such as nasal cancer, trauma, epistaxis, and diseases presenting with vascular disorders or mucosal disorders such as polyangiitis granulomatosa. Further, examples of a disease determined from an image of the vagina among the natural openings include tumors such as cervical cancer, diseases presenting with dryness, such as Sjogren's syndrome, diseases presenting with mucosal disorders and bleeding, such as vaginal erosions, trauma, inflammation such as vaginitis, infections such as vaginal candida, and diseases presenting with ulcers and skin lesions, such as a Behcet's disease. Further, examples of a disease determined from an image of the rectum among the natural openings include tumors such as rectal cancer, diseases such as ulcerative colitis that cause mucosal damage and bleeding, infections such as enteritis, and trauma.

Note that, in the present disclosure, terms such as "determination" and "diagnosis" for a disease are used, but these terms do not necessarily mean a definite determination or diagnosis by a doctor. For example, it is also possible to naturally include determination and diagnosis with the processing system 1 of the present disclosure used by the subject person himself/herself or used by an operator other than a doctor, or with the processing device 100 included in the processing system 1.

Further, in the present disclosure, the subject person to be imaged by the imaging device 200 can include any human such as a patient, an examinee, a subject person to be diagnosed, and a healthy person. Further, in the present disclosure, the operator who holds the imaging device 200 and performs imaging operation is not limited to a medical worker such as a doctor, a nurse, or a laboratory technician, and can include any human such as the subject person himself/herself. The processing system 1 according to the present disclosure is typically assumed to be used in a medical institution. However, the present disclosure is not limited to this case, and the place for using the processing system may be any place such as the subject person's home, school, or workplace.

Further, in the present disclosure, as described above, the subject may include at least a part of the natural opening of the subject person. Further, the disease to be determined may be any disease in which the differences appear in the findings in the natural opening which is the subject. However, in the following description, a case will be described in which the subject includes at least a part of the oral cavity, particularly the pharynx or a pharyngeal area, and the possibility of contracting the influenza as the disease is determined.

Further, in the present disclosure, the subject image may be one or a plurality of moving images or one or a plurality of still images. As examples of operations, if a power button is pressed down, a through image is fetched by a camera, and the captured through image is displayed on a display 203. Thereafter, if a capture button is pressed down by the operator, the one or the plurality of still images are captured by the camera, and the captured image is displayed on the display 203. Alternatively, if the capture button is pressed down by the subject person, capture of a moving image is started, and the image being captured by the camera during that period is displayed on the display 203. Then, if the capture button is pressed down again, the capture of the moving image ends. In this way, in a series of operations, various images such as the through image, the still image, and the moving image are captured by the camera and displayed on the display. However, the subject image does not refer to only a specific image among these images, but may include all of the images captured by the camera.

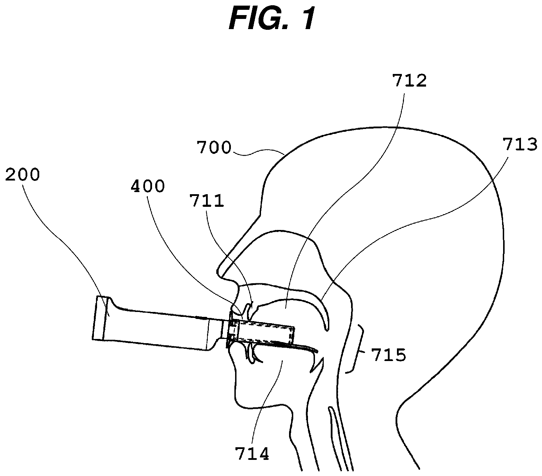

Fig. 1 is the view illustrating the use state of the imaging device 200 according to one embodiment of the present disclosure. According to Fig. 1, the operator attaches an assistance tool 400 in a manner that a distal end of the imaging device 200 is covered with the assistance tool, and inserts the imaging device 200 into an oral cavity 712 of a subject person 700 together with the assistance tool 400. Specifically, first, the operator (who may be the subject person 700 himself/herself or may be one different from the subject person 700) attaches the assistance tool 400 in the manner that the distal end of the imaging device 200 is covered with the assistance tool. Then, the operator inserts the imaging device 200 to which the assistance tool 400 is attached into the oral cavity 712. At this time, a distal end of the assistance tool 400 passes through an incisor 711 and is inserted to an area near a soft palate 713. In other words, the imaging device 200 is similarly inserted to the area near the soft palate 713. At this time, a tongue 714 is pushed downward by the assistance tool 400 (functioning as a tongue depressor), and movement of the tongue 714 is restricted. Accordingly, it is possible for the operator to secure a good field of view of the imaging device 200 and perform excellent imaging of a pharynx 715 located in front of the imaging device 200.

The captured subject image (typically, an image including the pharynx 715) is transmitted from the imaging device 200 to the server device 300 communicably connected via a wired or wireless network. A processor of the server device 300 that has received the subject image processes a program stored in a memory, to discriminate whether or not the subject image is an image suitable for determining the possibility of contracting a predetermined disease, and determine the possibility of contracting the predetermined disease from the subject image. Then, a result is transmitted to the processing device 100, and output to the display or the like via an output interface of the processing device 100.

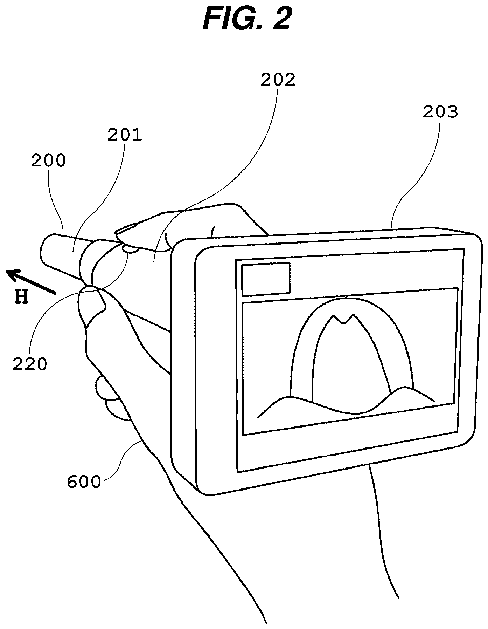

Fig. 2 is the view illustrating the use state of the imaging device 200 according to one embodiment of the present disclosure. Specifically, Fig. 2 is a view illustrating a state where an operator 600 holds the imaging device 200. According to Fig. 2, the imaging device 200 includes a main body 201, a grip 202, and the display 203 from a side to be inserted into the oral cavity. The main body 201 and the grip 202 are formed in a substantially columnar shape having a predetermined length along an insertion direction H into the oral cavity. Further, the display 203 is disposed on a side of the grip 202 opposite to the main body 201 side. Therefore, the imaging device 200 is entirely formed in a substantially columnar shape, and held by the operator 600 in a holding manner as holding a pencil. In other words, since a display panel of the display 203 faces a direction of the operator 600 in the use state, it is possible to easily handle the imaging device 200 while checking the subject image captured by the imaging device 200 in real time.

Further, when the operator 600 holds the grip 202 in a direction where the subject image is displayed in a normal direction on the display 203, a capture button 220 is configured to be disposed on an upper surface side of the grip. Therefore, when the operator 600 holds the grip, the operator 600 can easily press down the capture button 220 with an index finger or the like.

### 2. Configuration of Processing System 1

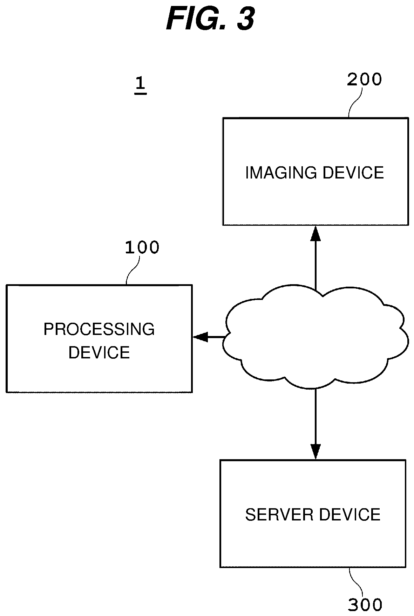

Fig. 3 is a schematic view of the processing system 1 according to one embodiment of the present disclosure. According to Fig. 3, the processing system 1 includes the processing device 100, the imaging device 200, and the server device 300, and the devices are communicably connected via the wired or wireless network. The processing device 100 inputs subject person information, interview information, finding information, and the like. Further, the processing device 100 outputs the subject image captured by the imaging device 200. Moreover, the processing device 100 receives a result of determining the possibility of contracting the predetermined disease based on the subject image captured by the imaging device 200 from the server device 300 and outputs the result.

The distal end of the imaging device 200 is inserted into the oral cavity of the subject person to image the oral cavity, particularly the pharynx. Specific imaging processes will be described later. The captured subject image is transmitted to the server device 300 via the wired or wireless network.

The server device 300 receives and manages the subject person information, the interview information, diagnosis information, and the like input in the processing device 100, and receives and manages the subject image captured in the imaging device 200. Further, the server device 300 discriminates whether or not the received the subject image is an image suitable for subsequent processes, transmits a result to the imaging device 200, determines the possibility of contracting the predetermined disease based on the subject image, the interview information, and the finding information, and transmits a result to the processing device 100.

Note that, in the present disclosure, the external device refers to the processing device 100, another processing device, the server device 300, another server device, or a combination thereof. In other words, unless otherwise specified, the external device may include any of the processing device 100, the server device 300, and a combination thereof.

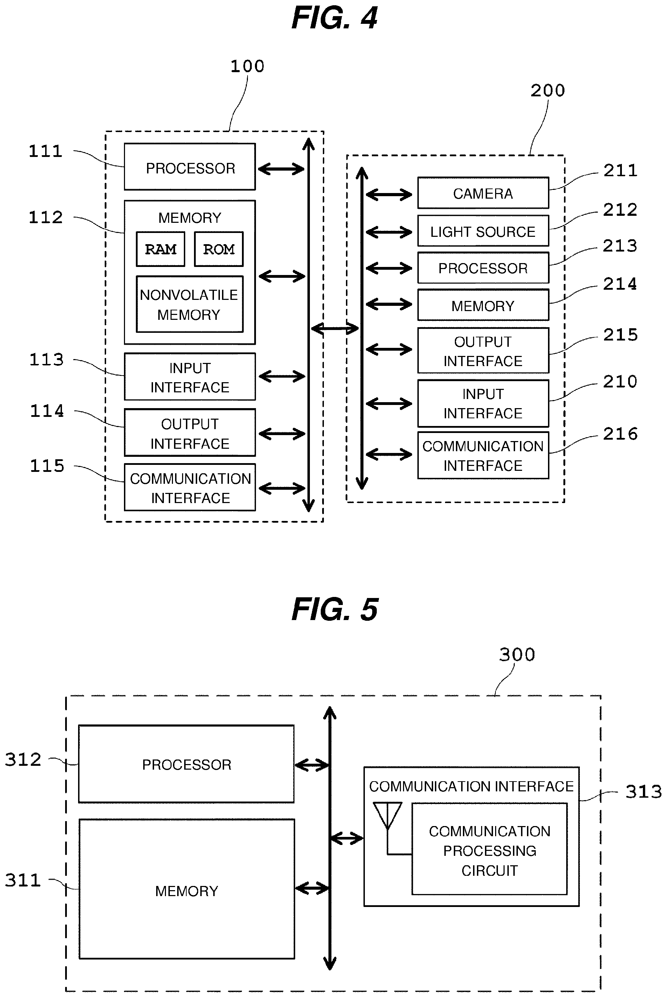

Fig. 4 is the block diagram illustrating the configurations of the processing device 100 and the imaging device 200 according to one embodiment of the present disclosure. Specifically, Fig. 4 is a view specifically illustrating the configurations of the processing device 100 and the imaging device 200 in the system 1. According to Fig. 4, the processing device 100 includes a processor 111, a memory 112, an input interface 113, an output interface 114, and a communication interface 115. Further, the imaging device 200 includes a camera 211, a light source 212, a processor 213, a memory 214, an output interface 215, an input interface 210, and a communication interface 216. These components are electrically connected to each other via a control line and a data line. Note that the processing device 100 and the imaging device 200 do not need to include all of the components illustrated in Fig. 4, and can also be configured without some of the components, or can also be configured by adding other components. For example, the processing device 100 and the imaging device 200 can include a battery for driving each of the components and the like. Note that, as described with reference to Fig. 3, the processing device 100 and the imaging device 200 only need to be communicably connected via the wired or wireless network, and are not necessarily configured to be directly communicable.

### 2-1. Configuration of Processing Device 100

First, in the processing device 100, the processor 111 functions as a control unit that controls other components of the processing system 1 based on a program stored in the memory 112. The processor 111 executes processes related to the input of the subject person information, the interview information, the finding information, and the like, the output of the subject image captured by the imaging device 200, and the output of the determination result of the possibility of contracting the predetermined disease based on the program stored in the memory 112. Specifically, the processor 111 executes "a process of receiving an input of the subject person information related to the subject person by the operator via the input interface 113", "a process of transmitting the received subject person information to the server device 300 via the communication interface 115", "a process of receiving an input of the interview information of the subject person by the operator or the subject person via the input interface 113", "a process of transmitting the received interview information to the server device 300 together with the subject person information via the communication interface 115", "a process of receiving the input of the subject person information related to the subject person by the operator via the input interface 113", "a process of transmitting the received subject person information to the server device 300 via the communication interface 115", "a process of receiving an input of the finding information of the subject person by the operator via the input interface 113", "a process of transmitting the received finding information to the server device 300 together with the subject person information via the communication interface 115", "a process of receiving the determination result indicating the possibility of contracting the predetermined disease determined based on the subject image or the like of the subject person and the subject image from the server device 300 via the communication interface 115, and outputting both the determination result and the subject image via the output interface 114", and the like based on the program stored in the memory 112. The processor 111 mainly includes one or a plurality of CPUs, and may be appropriately combined with GPU, an FPGA, or the like.

The memory 112 includes a RAM, a ROM, a nonvolatile memory, an HDD, and the like, and functions as a storage unit. The memory 112 stores instruction commands for various control operations of the processing system 1 according to the present embodiment as a program. Specifically, the memory 112 stores the program for the processor 111 to execute "the process of receiving the input of the subject person information related to the subject person by the operator via the input interface 113", "the process of transmitting the received subject person information to the server device 300 via the communication interface 115", "the process of receiving the input of the interview information of the subject person by the operator or the subject person via the input interface 113", "the process of transmitting the received interview information to the server device 300 together with the subject person information via the communication interface 115", "the process of receiving the input of the subject person information related to the subject person by the operator via the input interface 113", "the process of transmitting the received subject person information to the server device 300 via the communication interface 115", "the process of receiving the input of the finding information of the subject person by the operator via the input interface 113", "the process of transmitting the received finding information to the server device 300 together with the subject person information via the communication interface 115", "the process of receiving the determination result indicating the possibility of contracting the predetermined disease determined based on the subject image or the like of the subject person and the subject image from the server device 300 via the communication interface 115, and outputting both the determination result and the subject image via the output interface 114", and the like. In addition to the program, the memory 112 further stores subject person information, the subject image, the interview information, the finding information, and the like of the subject person.

The input interface 113 functions as an input unit that receives an instruction input from the operator to the processing device 100. Examples of the input interface 113 include physical key-buttons such as a "confirmation button" for performing various selection operations, a "return/cancel button" for returning to a previous screen or canceling a confirmation operation input, a cross key-button for moving a pointer or the like output to the output interface 114, an on/off key for turning on/off the power of the processing device 100, and a character input key-button for inputting various characters. Note that, as the input interface 113, it is also possible to use a touch panel disposed to be superimposed on the display functioning as the output interface 114 and having an input coordinate system corresponding to a display coordinate system of the display. In this case, icons corresponding to the above physical keys are displayed on the display, and the operator performs the instruction input via the touch panel to select each of the icons. A method of detecting the instruction input of the subject person by the touch panel may be any method such as a capacitance type or a resistive film type. In addition to the above, a mouse, a keyboard, or the like can also be used as the input interface 113. The input interface 113 does not always need to be physically provided in the processing device 100, and may be connected as necessary via the wired or wireless network.

The output interface 114 functions as an output unit for outputting information such as the determination result received from the server device 300. Examples of the output interface 114 include a display such as a liquid crystal panel, an organic EL display, or a plasma display. However, the processing device 100 itself does not necessarily include the display. For example, the interface for connecting to the display or the like connectable to the processing device 100 via the wired or wireless network can also function as the output interface 114 that outputs display data to the display or the like.

The communication interface 115 functions as a communication unit for transmitting and receiving the subject person information, the interview information, the subject image, the finding information, and the like to and from the server device 300 connected via the wired or wireless network. Examples of the communication interface 115 include various elements, for example, a connector for wired communication such as a USB and an SCSI, a transmission/reception device for wireless communication such as a wireless LAN, Bluetooth (registered trademark), and an infrared ray, and various connection terminals for a printed mounting board and a flexible mounting board.

### 2-2. Configuration of imaging device 200

In the imaging device 200, the camera 211 functions as an imaging unit that generates the subject image by detecting reflected light reflected on the oral cavity which is the subject. In order to detect the light, the camera 211 includes, as an example, a CMOS image sensor, a lens system, and a drive system for implementing a desired function. The image sensor is not limited to the CMOS image sensor, and other sensors such as a CCD image sensor can also be used. Although not particularly illustrated, the camera 211 can have an autofocus function, and it is preferable that a focus of the camera be set, for example, on the front of the lens to match a specific site. Further, the camera 211 can have a zoom function, and is preferably set to capture an image at an appropriate magnification according to a size of the pharynx or the influenza follicles.

Here, it has been known that there is the specific pattern for the influenza in the lymphatic follicles appearing at the deepest part of the pharynx located inside the oral cavity. The lymphatic follicles having this specific pattern are called influenza follicles, which are a characteristic sign of influenza, and are said to appear about 2 hours after onset. As described above, the processing system 1 of the present embodiment is used to determine the possibility of the subject person contracting the influenza by imaging the pharynx of the oral cavity and detecting the above follicles, for example. Therefore, if the imaging device 200 is inserted into the oral cavity, a distance between the camera 211 and the subject becomes relatively short. Accordingly, the camera 211 preferably has an angle of view (2θ) at which a value calculated by [(distance from the distal end portion of the camera 211 to a rear wall of the pharynx) * tanθ] is 20 mm or more in a vertical direction and 40 mm or more in a horizontal direction. By using the camera having such angle of view, even if the camera 211 and the subject are close to each other, it is possible to image a wider range. In other words, as the camera 211, a normal camera can be used, but a camera called a wide-angle camera or a super-wide-angle camera can also be used.

Further, in the present embodiment, a main subject imaged by the camera 211 is the influenza follicles formed in the pharynx or a pharynx portion. Since the pharynx is generally formed deep in a depth direction, if a depth of field is shallow, the focus is shifted between an anterior part of the pharynx and the posterior part of the pharynx, and it becomes difficult to obtain the subject image suitable for use in the determination in the processing device 100. Accordingly, the camera 211 has a depth of field of at least 20 mm or greater, preferably 30 mm or greater. By using the camera having such depth of field, it is possible to obtain the subject image having a focus at any site from the anterior part of the pharynx to the posterior part of the pharynx.

The light source 212 is driven by an instruction from the processor 213 of the imaging device 200, and functions as a light source unit for irradiating the oral cavity with the light. The light source 212 includes one or more light sources. In the present embodiment, the light source 212 includes one or a plurality of LEDs, and light having a predetermined frequency band is emitted from each of the LEDs in a direction of the oral cavity. As the light source 212, light having a desired band among an ultraviolet light band, a visible light band, and an infrared light band, or a combination thereof is used. Note that, in a case where the possibility of contracting the influenza is determined in the processing device 100, it is preferable to use light in the visible light band.

The processor 213 functions as a control unit that controls other components of the imaging device 200 based on the program stored in the memory 214. Based on the program stored in the memory 214, the processor 213 executes "a process of receiving the subject person information including first subject person information associated with one or each of a plurality of subject persons including a first subject person via the communication interface 216 from the external device (the processing device 100 or the server device 300) communicably connected to the imaging device 200 via a network", "a process of, in a case of outputting, as a list, pieces of subject person information of unimaged subject persons among the one or the plurality of subject persons, outputting the list without including the first subject person information before the first subject person information associated with the first subject person is registered in the external device, and outputting the list including the first subject person information after the first subject person information is registered in the external device", "a process of outputting attribute information of the first subject person when selection of the first subject person information is received from the list output including the first subject person information", "a process of, after receiving the selection of the first subject person information from the list output including the first subject person information, transmitting the first subject person information and a first subject image to the external device in association with each other when the first subject image including at least a part of an oral cavity of the first subject person is imaged by the camera", "a process of receiving discrimination information indicating whether or not the first subject image is appropriate for use in the determination from the external device, and outputting the received discrimination information", "a process of outputting an attachment indication that promotes attachment of the assistance tool 400 which covers at least a part of the imaging device and is inserted into the oral cavity together with a part of the assistance tool", and the like based on the program stored in the memory 214. The processor 213 mainly includes the one or the plurality of CPUs, and may be appropriately combined with the GPU, the FPGA, or the like.

The memory 214 includes the RAM, the ROM, the nonvolatile memory, the HDD, and the like, and functions as the storage unit. The memory 214 stores the instruction commands for various control operations of the processing system 1 according to the present embodiment as the program. Specifically, the memory 214 stores the program for the processor 213 to execute "the process of receiving the subject person information including first subject person information associated with the one or each of the plurality of subject persons including the first subject person via the communication interface 216 from the external device (the processing device 100 or the server device 300) communicably connected to the imaging device 200 via the network", "the process of, in the case of outputting, as a list, the pieces of the subject person information of the unimaged subject persons among the one or the plurality of subject persons, outputting the list without including the first subject person information before the first subject person information associated with the first subject person is registered in the external device, and outputting the list including the first subject person information after the first subject person information is registered in the external device", "a process of outputting the attribute information of the first subject person when the selection of the first subject person information is received from the list output including the first subject person information", "the process of, after receiving the selection of the first subject person information from the list output including the first subject person information, transmitting the first subject person information and the first subject image to the external device in association with each other when the first subject image including at least the part of the oral cavity of the first subject person is imaged by the camera", "the process of receiving the discrimination information indicating whether or not the first subject image is appropriate for use in the determination from the external device, and outputting the received discrimination information", "the process of outputting the attachment indication that promotes the attachment of the assistance tool 400 which covers at least the part of the imaging device and is inserted into the oral cavity together with the part of the assistance tool", and the like. In addition to the program, the memory 214 further stores the subject person information, the subject image, and the like of the subject person.

The output interface 215 functions as an output unit for outputting the subject image, the subject person information, and the like captured by the imaging device 200. Examples of the output interface 215 include the display 203, but is not limited thereto, and may include another liquid crystal panel, the organic EL display, or the plasma display. Further, the display 203 is not necessarily included, for example, the interface for connecting to the display or the like connectable to the processing device 100 via the wired or wireless network can also function as the output interface 114 that outputs display data to the display or the like.

The input interface 210 functions as an input unit that receives an instruction input from the operator to the imaging device 200. Examples of the input interface 210 include physical key-buttons such as a "capture button" for instructing start/end of recording by the imaging device 200, a "power button" for turning on/off the power of the imaging device 200, a "confirmation button" for performing various selection operations, a "return/cancel button" for returning to a previous screen or canceling an input confirmation operation, and a cross key-button for moving an icon or the like displayed on the output interface 215. Note that these various buttons/keys may be physically prepared, or may be selectable using a touch panel or the like displayed as an icon on the output interface 215 and arranged as the input interface 210 in a superimposed manner on the output interface 215. A method of detecting the instruction input of the subject person by the touch panel may be any method such as a capacitance type or a resistive film type.

The communication interface 216 functions as a communication unit for transmitting and receiving information to and from the server device 300 and/or other devices. Examples of the communication interface 216 include various elements, for example, the connector for wired communication such as the USB and the SCSI, the transmission/reception device for wireless communication such as the wireless LAN, the Bluetooth (registered trademark), and the infrared ray, and various connection terminals for the printed mounting board and the flexible mounting board.

### 2-3. Configuration of Server Device 300

Fig. 5 is the block diagram illustrating the configuration of the server device 300 according to one embodiment of the present disclosure. According to Fig. 5, the server device 300 includes a memory 311, a processor 312, and a communication interface 313. These components are electrically connected to each other via a control line and a data line. Note that the server device 300 does not need to include all of the components illustrated in Fig. 5, and can also be configured without some of the components, or can also be configured by adding other components. For example, it is also possible to configure the server device 300 integrally by being connected to another server device. Further, it is also possible to configure the server device 300 integrally by being connected to another database device.

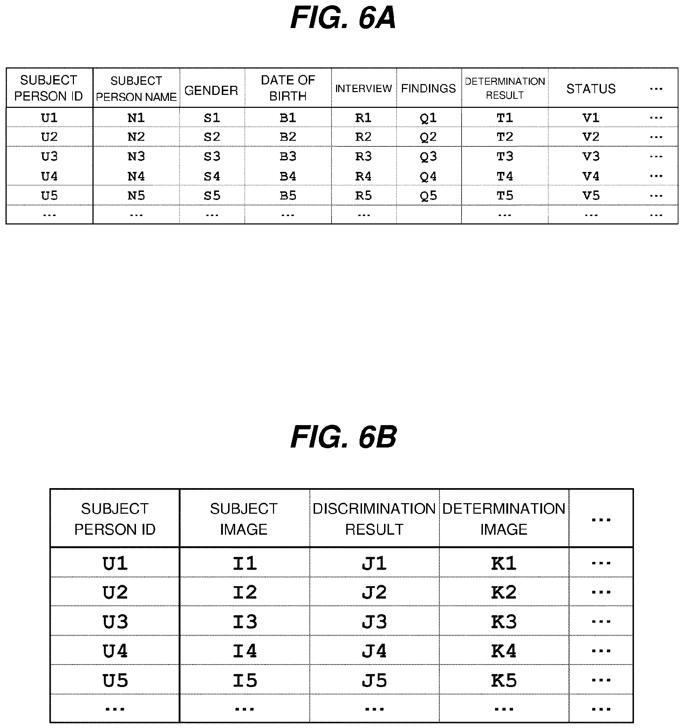

The memory 311 includes the RAM, the ROM, the nonvolatile memory, the HDD, and the like, and functions as the storage unit. The memory 311 stores the instruction commands for various control operations of the processing system 1 according to the present embodiment as the program. Specifically, the memory 311 stores the program for the processor 312 to execute "a process of receiving the subject person information received by the processing device 100 from the processing device 100 via the communication interface 313", "a process of storing the received subject person information in the subject person management table in association with the subject person ID information", "a process of receiving the subject person information request from the imaging device 200 via the communication interface 313, and extracting the subject person information of the subject person for whom the imaging of the inner side of the oral cavity has not yet been completed with reference to the subject person management table", "a process of transmitting the extracted subject person information of the unimaged subject person to the imaging device 200 via the communication interface 313", "a process of receiving subject images captured by the imaging device 200 from the imaging device 200 via the communication interface 313 and storing the subject images in the image management table in association with the subject person ID information", "a process of discriminating whether or not the received subject images are the images suitable for determining the possibility of contracting the predetermined disease", "a process of storing a discrimination result in the image management table if the discrimination result is determined to be appropriate and transmitting the discrimination result to the processing device 100 and the imaging device 200 via the communication interface 313", "a process of receiving the request from the processing device 100 and transmitting the subject images and information for specifying an image to be used as the determination image among the subject images to the processing device 100 via the communication interface 113", "a process of receiving the interview information and the finding information input by the processing device 100 from the processing device 100 via the communication interface 313, and storing the information in the subject person management table in association with the subject person ID information", "a process of, upon receiving a determination request for the possibility of contracting the predetermined disease of the subject person selected in the processing device 100 from the processing device 100 via the communication interface 313, reading out the subject images associated with the subject person from the image management table and the interview information and the subject person information associated with the subject person from the subject person management table, and determining the possibility", "a process of transmitting the determined result to the processing device 100 via the communication interface 313", and the like. In addition to the program, the memory 311 stores various types of information stored in the subject person management table (Fig. 6A) and the image management table (Fig. 6B), image data itself such as the subject image, and the like.

The processor 312 functions as a control unit that controls other components of the server device 300 based on the program stored in the memory 311. Based on the program stored in the memory 311, the processor 312 performs a process of discriminating whether or not the image is appropriate for determining the possibility of contracting the predetermined disease, and a process of determining the possibility of contracting the predetermined disease. Specifically, the processor 312 executes "the process of receiving the subject person information received by the processing device 100 from the processing device 100 via the communication interface 313", "the process of storing the received subject person information in the subject person management table in association with the subject person ID information", "the process of receiving the subject person information request from the imaging device 200 via the communication interface 313, and extracting the subject person information of the subject person for whom the imaging of the inner side of the oral cavity has not yet been completed with reference to the subject person management table", "the process of transmitting the extracted subject person information of the unimaged subject person to the imaging device 200 via the communication interface 313", "the process of receiving subject images captured by the imaging device 200 from the imaging device 200 via the communication interface 313 and storing the subject images in the image management table in association with the subject person ID information", "the process of discriminating whether or not the received subject images are the images suitable for determining the possibility of contracting the predetermined disease", "the process of storing the discrimination result in the image management table if the discrimination result is determined to be appropriate and transmitting the discrimination result to the processing device 100 and the imaging device 200 via the communication interface 313", "the process of receiving the request from the processing device 100 and transmitting the subject images and the information for specifying the image to be used as the determination image among the subject images to the processing device 100 via the communication interface 113", "the process of receiving the interview information and the finding information input by the processing device 100 from the processing device 100 via the communication interface 313, and storing the information in the subject person management table in association with the subject person ID information", "the process of, upon receiving the determination request for the possibility of contracting the predetermined disease of the subject person selected in the processing device 100 from the processing device 100 via the communication interface 313, reading out the subject images associated with the subject person from the image management table and the interview information and the subject person information associated with the subject person from the subject person management table, and determining the possibility", "the process of transmitting the determined result to the processing device 100 via the communication interface 313", and the like, based on the program stored in the memory 311. The processor 312 mainly includes the one or the plurality of CPUs, but may be appropriately combined with the GPU, the FPGA, or the like.

### 3. Information Stored in Memory 311 of Server Device 300

Fig. 6A is a view conceptually illustrating the subject person management table stored in the server device 300 according to one embodiment of the present disclosure. The information stored in the subject person management table is updated and stored as needed in accordance with progress of the processes of the processor 312 of the server device 300.

According to Fig. 6A, the subject person management table stores subject person name information, gender information, date of birth information, the interview information, the finding information, determination result information, status information, and the like in association with the subject person ID information. The "subject person ID information" is information unique to each subject person, for specifying each subject person. The subject person ID information is generated every time a new subject person is registered by the operator or the subject person himself/herself. The "subject person name information" is information indicating a name of each subject person output from the processing device 100 or the imaging device 200, and is information indicating any character string input by the operator or the subject person himself/herself. The "gender information" is information indicating a gender of the subject person, and is information selected by the operator or the subject person himself/herself. Preferably, a biological gender of each subject person is stored. The "date of birth information" is information indicating a date of birth of the subject person. The information is not limited to a year, a month, and a day, but may be information of only the year, and age information or the like can be used instead of the date of birth information.

The "interview information" is, for example, information input by the operator, the subject, or the like, and is information such as the subject person's medical history and symptoms that is used as a reference for diagnosis by a doctor. Examples of such interview information include patient background such as a body weight, an allergy, and a basal disease, a body temperature, a peak body temperature from onset, elapsed time from the onset, a heart rate, a pulse rate, an oxygen saturation, a blood pressure, a medication administration status, a contact status with other influenza patients, joint pain, muscle pain, headache, malaise, loss of appetite, chills, sweating, cough, sore throat, nasal juice/nasal congestion, tonsillitis, digestive symptoms, rash on hands and feet, redness and white moss of the pharynx, swelling of the tonsils, history of resection of the tonsils, presence or absence of subjective symptoms and physical findings such as a strawberry tongue and swelling of an anterior cervical lymph node with tenderness, history of influenza vaccination, and vaccination time. The "finding information" is information input by the operator such as the doctor, and is information indicating a state different from a normal state obtained by a test for assisting various types of examination and diagnose of inspection, interview, palpation, and auscultation of the subject person. Examples of such finding information include the redness and the white moss of the pharynx, the swelling of the tonsils, the presence or absence of the tonsillitis, redness and white moss of the tonsils, and the like.

The "determination result information" is information indicating a determination result of the possibility of contracting the influenza determined based on the interview information, the finding information, and the determination image. An example of such determination result information is a positive rate for the influenza. However, the information is not limited to the positive rate, and any information that indicates the possibility, such as information specifying a positive or negative result, is acceptable. Further, the determination result does not need to be a specific numerical value, and may be in any form such as a classification according to the positive rate or a classification indicating the positive or negative result. The "status information" is information indicating a current status of each subject person. As such status information, stored are information such as "interview not completed" indicating that input of the interview information has not yet been completed, "unimaged" indicating that the capture of the subject image has not yet been completed (that is, acquisition of the determination image has not been performed), "imaged" indicating that input of the findings has not been completed by a doctor or the like although the imaging of the subject image has been completed (that is, the acquisition of the determination image has been performed), and "determined" indicating that the input of the findings has been completed and the possibility of contracting the predetermined disease has been determined. Note that such status information is merely an example, and the status can also be defined in more detail, or the status can also be defined more broadly.

Note that information illustrated in Fig. 6A is information stored as the subject person information. Further, among pieces of the information, information other than the subject person ID information is used as the attribute information. Further, in Fig. 6A, the attribute information is stored in association with the subject person ID information, but it is not necessary to store all the information stored here, and for example, information other than the information described here, such as personal information about the subject person, for example, an address, a telephone number, a family composition, and current epidemic information on the infectious diseases such as the influenza.

Further, the interview information and the finding information are not necessarily input by the subject person or the operator via the processing device 100 or the like each time, and may be received from, for example, an electronic medical record device, another terminal device, or the like connected via the wired or wireless network. Further, the information may be acquired by analyzing the subject image captured by the imaging device 200. Moreover, although not particularly illustrated in Figs. 6A and 6B, it is also possible to further store, in the memory 112, the current epidemic information on the infectious diseases that are targets for diagnosis or assistance in the diagnosis, such as the influenza, and external factor information such as a determination result and a disease condition of another subject person regarding these infectious diseases.

Fig. 6B is the view conceptually illustrating the image management table stored in the server device 300 according to one embodiment of the present disclosure. The information stored in the image management table is updated and stored as needed in accordance with the progress of the processes of the processor 312 of the server device 300.

According to Fig. 6B, subject image information, discrimination result information, determination image information, and the like are stored in the image management table in association with the subject person ID information. The "subject person ID information" is the information unique to each subject person, for specifying each subject person. The "subject image information" is information for specifying the subject image captured by the operator for each user. The subject image is one or a plurality of images including the subject imaged by the camera of the imaging device 200, and is stored in the memory 112 by being received from the imaging device 200. The "discrimination result information" is information indicating a result of determining whether or not the received subject image is an image suitable for determining the possibility of contracting the predetermined disease. The "determination image information" is information for specifying the determination image used for determining the possibility of contracting the predetermined disease. Note that, as described above, information for specifying each image is stored as the subject image information. In this way, the information for specifying the image is typically identification information for identifying each image, and may be information indicating a storage location of each image.

### 4. Processing Sequences Executed by Processing device 100 and Imaging Device 200

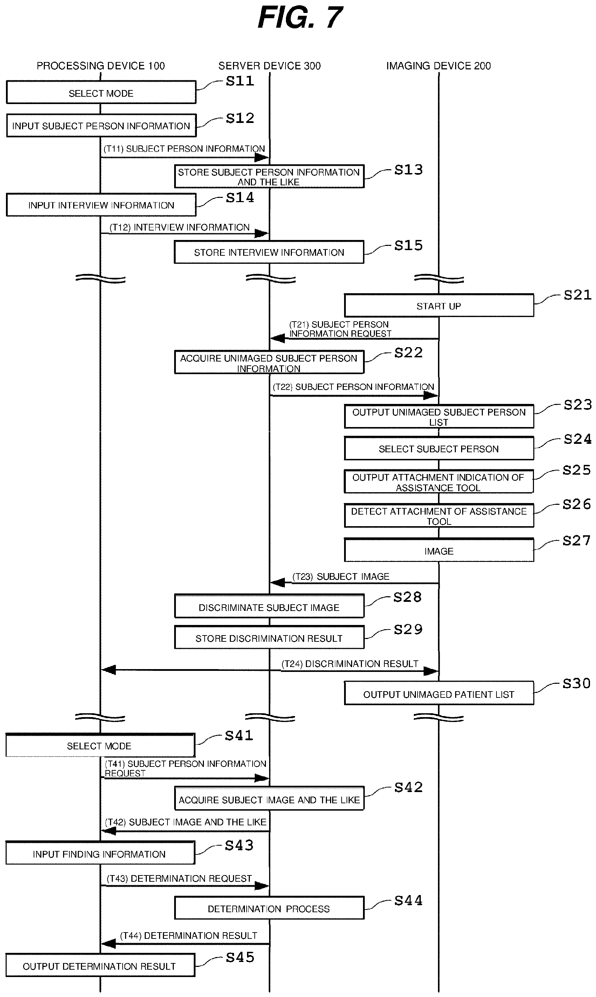

Fig. 7 is the view illustrating the processing sequences to be executed among the processing device 100, the imaging device 200, and the server device 300 according to one embodiment of the present disclosure. Among the steps, S11 to S14 mainly indicate a processing sequence related to an input process such as the subject person information performed between the processing device 100 and the server device 300, S21 to S30 mainly indicate a processing sequence related to imaging processes performed between the imaging device 200 and the server device 300, and S41 to S46 mainly indicate a processing sequence related to a determination process performed between the processing device 100 and the server device 300. Note that, in the following description, for convenience of description, an example will be described in which the "first subject person" is newly registered as a subject person as one of the subject persons and the possibility of the contraction is determined.

### 4-1. Input Process of Subject Person Information and the Like

According to Fig. 7, the processing device 100 receives selection of a new registration mode for newly registering the subject person via the input interface 113 on a subject person list screen output via the output interface 114 (S11). Note that, here, description of various screens output before the subject person list screen is omitted. Then, when outputting a new registration screen on the display via the output interface 114, the processing device 100 outputs newly generated subject person ID information, and receives an input of the subject person name information, the gender information, and the date of birth information of the first subject person via the input interface 113 (S12). Then, the processing device 100 transmits the received subject person information (T11) to the server device 300 via the communication interface 115 in association with the subject person ID information. When receiving the subject person information of the first subject person via the communication interface 313, the server device 300 stores the subject person information in the subject person management table in the memory 311 in association with the subject person ID information received together (S13).

Next, when outputting an interview information input screen of the first subject person to the display via the output interface 114, the processing device 100 receives inputs of various pieces of interview information such as symptoms for the predetermined disease, and the patient background such as the body weight, the allergy, and the basal disease via the input interface 113 (S14). The processing device 100 transmits the received interview information (T12) to the server device 300 via the communication interface 115 in association with the subject person ID information. When receiving the interview information of the first subject person via the communication interface 313, the server device 300 stores the interview information in the subject person management table in the memory 311 in association with the subject person ID information received together (S15). At this time, since the server device 300 has received the subject person information and the interview information as the status information, "unimaged" indicating that the imaging of the subject image has not yet been completed (that is, the acquisition of the determination image is not performed) is stored. In this way, the processing sequence related to the input process of a series of the subject person information and the like ends. In this way, the interview information is input and transmitted in the processing device 100 in association with the subject person ID information for specifying the subject person, and is stored in the server device 200. Therefore, it is possible to accurately associate and manage a correspondence relationship between the input interview information and the subject person.

Note that, in Fig. 7, the interview information is input at a timing of S14, but this input may be performed at any timing. For example, the input can also be performed after the imaging processes and before the input of the finding information. Further, the input of the subject person information (S12) and the input of the interview information (S14) are received as separate processes, but after the subject person information of the subject person is input, an interview input screen of the subject person may be displayed without change to receive the input of the interview information.

### 4-2. Imaging Processes

According to Fig. 7, the power of the imaging device 200 is turned on by pressing down the power button or the like, and the imaging device 200 is started up (S21). When being started up, the imaging device 200 transmits the subject person information request (T21) to the server device 300 via the communication interface 216.

When receiving the subject person information request (T21), the server device 300 searches for subject persons "unimaged" indicating that the capture of the subject image has not yet been completed with reference to the status information in the subject person management table. Then, the server device 300 acquires the subject person information of each of the subject persons for which "unimaged" is stored as the status information, including the first subject person (S22). The server device 300 transmits the subject person information (T22) of each of the unimaged subject persons including the first subject person to the imaging device 200 via the communication interface 313.

When receiving the subject person information via the communication interface 216, the imaging device 200 outputs, as a list, the unimaged subject persons including the first subject person to the display via the output interface 215 (S23). When receiving the selection of the subject person information of a subject person (here, the first subject person is taken as an example) to be imaged from the output list via the input interface 210 (S24), the imaging device 200 outputs attribute information of the first subject person to the display via the output interface 215. In this way, by outputting the attribute information of the first subject person to be imaged, the subject person ID information of the first subject person and the subject image to be captured thereafter can be reliably associated with each other, and it is possible to prevent mistaking of the subject person and the like.

The imaging device 200 determines whether or not the assistance tool 400 is attached, and in a case where the assistance tool has not yet been attached, attaches an attachment indication for prompting the attachment via the output interface 215 (S25). Note that the indication is merely an example, and the attachment may be promoted in other manners such as blinking of sound or light, vibration, or the like. Then, when it is detected that the assistance tool is attached to the imaging device 200 (S26), the imaging device 200 captures the subject image (S27). When capturing the subject image, the imaging device 200 transmits the subject image (T23) captured via the communication interface 216 to the server device 300 together with the subject person ID information of the first subject person. Note that, in S26, the attachment of the assistance tool 400 is detected, but the process itself may be skipped. Further, instead of detecting the attachment of the assistance tool 400, it may also be possible for the operator himself/herself to input that the assistance tool 400 has been attached by, for example, making it possible to output a check indication for the operator to check the attachment of the assistance tool 400 via the output interface 215, and receive a predetermined operation input (for example, a tap operation) for the check indication by the operator.

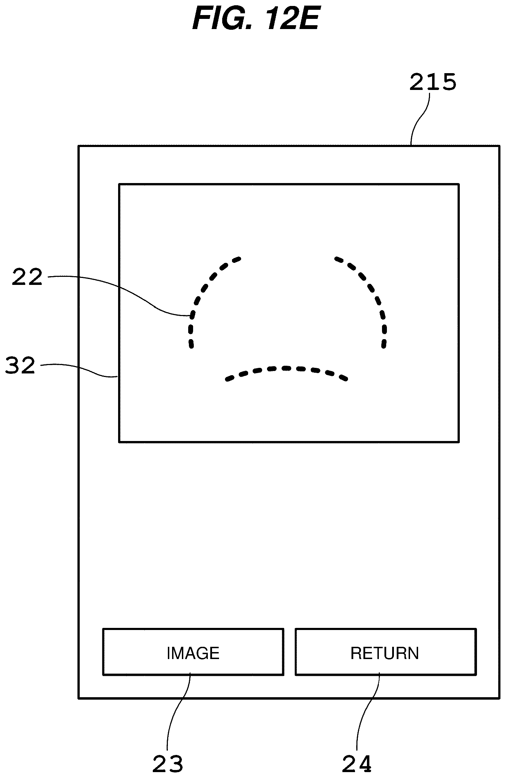

When receiving the subject image via the communication interface 313, the server device 300 stores the received subject image in the image management table in association with the subject person ID information received together, and stores image data of the received subject image in the memory 311. Then, the server device 300 discriminates whether or not he received subject image is the image suitable for determining the possibility of contracting the predetermined disease (S28). Then, in a case where there is no suitable image at all, the server device 300 transmits a notification for prompting reimaging to the imaging device 200 (not illustrated). Meanwhile, in a case where there is a suitable image (for example, one or a plurality of subject images having a highest similarity to the suitable image), the server device 300 stores the image as an image that can be used as the determination image in the determination image information of the image management table in association with the subject person ID information of the first subject person (S29). Further, the server device 300 updates the status information associated with the subject person ID information of the first subject person to "imaged" with reference to the subject person management table, and transmits information (T24) indicating that the determination image has been obtained as a discrimination result to the processing device and the imaging device 200 via the communication interface 313 together with the subject person ID information of the first subject person. Note that, although not particularly illustrated, as illustrated in Fig. 12E, the discrimination result may be transmitted to the imaging device 200, and the operation input by the operator may be received to select whether or not to confirm the determination image. In other words, an image discriminated to be usable as the determination image may be previewed once on the imaging device 200, and the determination image may be finally confirmed by the server device 300 receiving that the confirmation operation has been performed and stored the determination image in the image management table.

When receiving the discrimination result via the communication interface 216, the imaging device 200 outputs the result via the output interface 215. Further, the pieces of the subject person information of the unimaged subject persons are received together with the discrimination result, and output the pieces of the subject person information as a list on the display via the output interface 215 as in S23 (S30). At this time, since the status information of the first subject person is "imaged", the status information is not included in the subject person information of the above unimaged subject person. Therefore, the list does not include the subject person information of the first subject person. In this way, the processing sequence related to a series of imaging processes ends.

### 4-3. Determination Process

According to Fig. 7, the processing device 100 receives, via the input interface 113 on the subject person list screen output via the output interface 114, selection of a subject person (here, the first subject person is taken as an example) to be diagnosed according to the subject person information for which "imaged" is displayed as the status information, and receives selection of a diagnosis mode for performing the diagnosis on the subject person (S41). Note that, here, description of various screens output before the subject person list screen is omitted. Then, the processing device 100 transmits the subject person information request (T41) for the selected first subject person to the server device 300 via the communication interface 115.

When receiving the subject person information request for the first subject person via the communication interface 313, the server device 300 acquires the attribute information of the first subject person based on the subject person ID information of the first subject person with reference to the subject person management table, and acquires the subject image associated with the subject person ID information of the first subject person and the determination image information in which the image to be used as the determination image is specified thereafter with reference to the image management table (S42). Then, the server device 300 transmits the acquired subject image, determination image information, and attribute information (T42) together with the subject person ID information of the first subject person to the processing device 100 via the communication interface 313.

When receiving the subject image, the determination image information, and the like via the communication interface 115, the processing device 100 outputs the received subject image, determination image information, and attribute information to the display via the output interface 114. Then, the processing device 100 receives the input of the finding information by the operator such as a doctor via the input interface 113 (S43). When the finding information is input, the processing device 100 transmits the determination request (T43) for the possibility of contracting the predetermined disease based on the interview information, the finding information, and the determination image together with the subject person ID information of the first subject person via the communication interface 115.

In this way, the server device 300 acquires the attribute information, the subject image, and the determination image information of the first subject person in association with the subject person ID information for specifying the subject person, and the processing device 100 outputs these pieces of information in association with the subject person ID information before inputting the finding information and transmitting the determination request. In this way, it is possible to reduce a risk of the mistaking of the subject person such as inputting the finding information or making the determination request for a wrong subject person. Further, in the server device 300, it is possible to reliably associate each subject person with the finding information and the determination result.

When receiving the determination request from the processing device 100 via the communication interface 313, the server device 300 stores the received finding information in the finding information of the subject person management table in association with the subject person ID information of the first subject person, and updates the status information. Then, the server device 300 performs the determination process of the possibility of contracting the predetermined disease based on the stored interview information, finding information, and determination image (S44). Details of the determination process will be described later. Note that, in the determination process, the determination may be performed only with the determination image without using the interview information and the finding information. When the determination process is made, the server device 300 stores the determination result information in the subject person management table based on the subject person ID information of the first subject person, and updates the status information to "determined". The server device 300 transmits the determination result information (T44) stored in the subject person management table to the processing device 100 together with the subject person ID information of the first subject person via the communication interface 313.

When receiving the determination result information via the communication interface 115, the processing device 100 outputs the received determination result to the display via the output interface 114 (S45). In this way, the processing sequence related to a series of imaging processes ends. As described above, according to the processing sequence, the subject person information, the interview information, the finding information, the subject image, and the determination result are acquired by different devices (the processing device 100, the imaging device 200, the server device 300, or the like) at different timings. However, in S12, first, these pieces of information are acquired after the subject person information is newly registered, and the subject person ID information is associated and processed during the acquirement of these pieces of information. Therefore, it is possible to accurately associate each subject person with the subject person information, the interview information, the finding information, the subject image, and the determination result, and it is possible to reduce the risk of the mistaking between the subject person and these pieces of information.

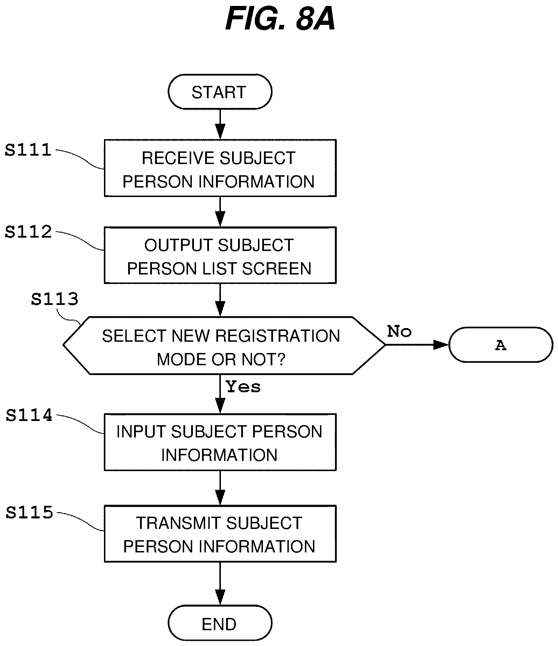

Note that, although the same applies to the processing flow illustrated in Fig. 8A and subsequent drawings, in Fig. 7, the input of the interview information is performed by the input process of the subject person information or the like, but the present disclosure is not limited to this timing, and may be performed at any timing such as during the imaging processes or during the determination process. Further, it is not necessary to input the information by the processing device 100, and for example, the interview information may be input via a website in advance or in a course of a series of the processes in a terminal device of the subject person possessed by the subject person. Further, in the determination process, the subject image is transmitted to the processing device 100, but only the image used as the determination image may be transmitted and output in the processing device 100. Further, in an example of Fig. 7, a case where the first subject person is registered and determined has been described, but even a subject person other than the first subject person can be similarly processed. Further, in the example of Fig. 7, the determination image is acquired by performing the discrimination process on the subject image, but the discrimination process of determining the possibility of the contraction may be performed using all or a part of the subject image acquired without performing the determination process.

### 5. Processing Flow to Be Executed by Processing Device 100

Fig. 8A is the chart illustrating the processing flow to be executed in the processing device 100 according to one embodiment of the present disclosure. Specifically, Fig. 8A is a chart illustrating a processing flow executed in a new registration process of the subject person information in S11 and S12 in the input process of the subject person information and the like in Fig. 7. The processing flow is mainly performed by the processor 111 of the processing device 100 reading and executing the program stored in the memory 112. Note that, although the first subject person will be described below as an example, other subject persons can be similarly processed.

According to Fig. 8A, the processor 111 accesses the server device 300 at a predetermined interval, and receives the subject person information stored in the subject person management table from the server device 300 (S111). Then, the processor 111 updates and outputs the subject person list screen output to the display via the output interface 114 based on the received subject person information (S112). At this time, since the subject person information of the first subject person has not yet registered, the first subject person is not output on the subject person list screen. Note that the reception of the subject person information in S111 is not limited to a case of being performed at a predetermined interval, and may be performed by receiving the instruction input of the operator.

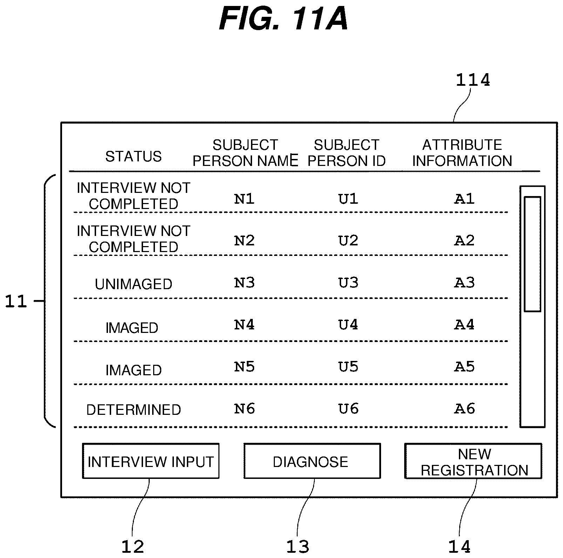

Here, Fig. 11A is the view illustrating the example of the screen to be displayed on the processing device 100 according to one embodiment of the present disclosure. Specifically, Fig. 11A is a view illustrating an example of the subject person list screen output from the processing device 100. According to Fig. 11A, the subject person list screen includes a list area 11 in which the received subject person information is output line by line for each subject person. Each row of the list area 11 includes the status information, the subject person name information, the subject person ID information, and the attribute information of each subject person. Further, although not particularly illustrated, when the input of the operator is received via the input interface 113 and a desired subject person to be processed is selected, a row in which the subject person information of the subject person is output is highlighted and displayed. As an example, in a case where a touch sensor is included as the input interface 113, the subject person is selected as a processing target by receiving an input of a tap operation on the subject person name of the desired subject person. Note that, since the highlight display described above is for notifying of the subject person information of the selected subject person, as long as the selected subject person can be identified, the notification may naturally be made by other methods.

Further, according to Fig. 11A, the subject person list screen includes an interview input icon 12, a diagnosis icon 13, and a new registration icon 14 below the list area 11. When selection of any icon is received via the input interface 113, a mode corresponding to the selected icon is activated. In other words, the mode shifts to an interview input mode in a case where the interview input icon 12 is selected, the mode shifts to the diagnosis mode in a case where the diagnosis icon 13 is selected, and the mode shifts to the new registration mode for the subject person in a case where the new registration icon 14 is selected.

Returning to Fig. 8A again, when receiving the selection of the new registration icon 14 illustrated in Fig. 11A via the input interface 113 (S113), the processor 111 outputs the new registration screen to the display via the output interface 114.

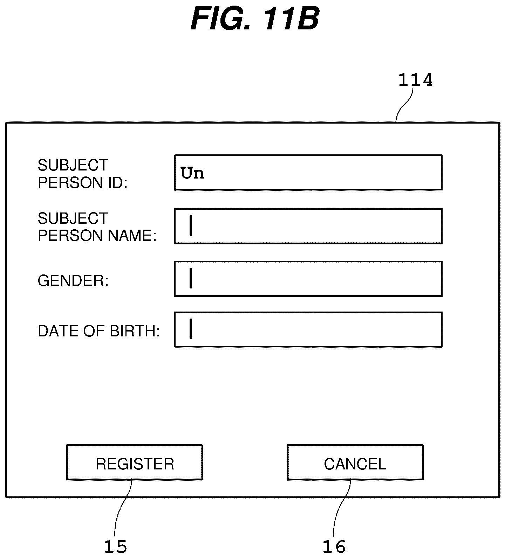

Here, Fig. 11B is the view illustrating the example of the screen to be displayed on the processing device 100 according to one embodiment of the present disclosure. Specifically, Fig. 11B is a view illustrating an example of the new registration screen of the subject person output from the processing device 100. According to Fig. 11B, the subject person ID information (Un) assigned in advance to the newly registered subject person is output on the new registration screen. Then, input boxes for inputting the subject person name information, the gender information, and the date of birth information required to be registered as the subject person information are output below the subject person ID information, respectively. When any of the input boxes is selected by the operator or the subject person, input of a desired character and the like and selection input of the information can be performed in the input boxes via the input interface 113.

Further, according to Fig. 11B, a registration icon 15 and a cancel icon 16 are output below each of the input boxes. When selection of the registration icon 15 is received via the input interface 113, the subject person information input to each of the input boxes is confirmed and transmitted to the server device 300. Meanwhile, when selection of the cancel icon 16 is received via the input interface 113, a new input of the subject person information ends at this point, and the screen returns to the subject person list screen again.

Returning to Fig. 8A again, the processor 111 receives the input of the subject person information of the first subject person illustrated in Fig. 11B via the input interface 113 (S114). Then, when receiving the selection of the registration icon 15 illustrated in Fig. 11B via the input interface 113, the processor 111 transmits the input subject person information to the server device 300 via the communication interface 115 in association with the subject person ID information (S115). In this way, the processor 111 ends the processing related to new registration of the subject person information of the first subject person.

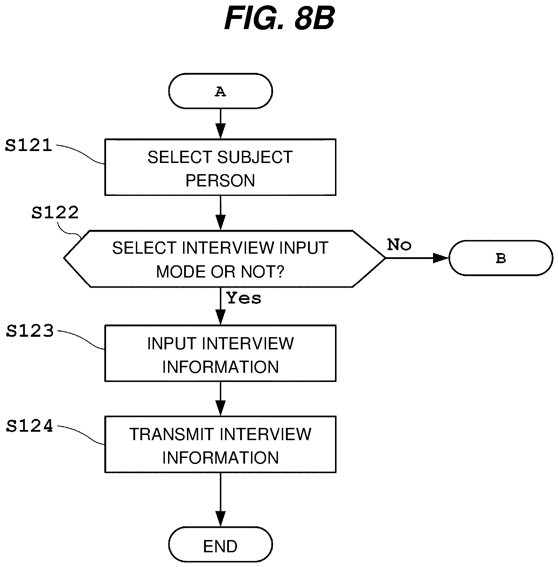

Fig. 8B is the chart illustrating the processing flow executed in the processing device 100 according to one embodiment of the present disclosure. Specifically, Fig. 8B is a chart illustrating a processing flow executed in the input process of the interview information in S13 in the input process of the subject person information and the like in Fig. **7****.** The processing flow is mainly performed by the processor 111 of the processing device 100 reading and executing the program stored in the memory 112. Note that, although the first subject person will be described below as an example, other subject persons can be similarly processed.

According to Fig. 8B, the processor 111 receives, via the input interface 113, selection of a desired subject person (the first subject person) who is to input the interview information on the subject person list screen (S112 in Fig. 8A) (S121). No that since the first subject person is newly registered as illustrated in Fig. 8A, the subject person information of the first subject person is included and displayed on the subject person list screen. Then, when the subject person name of the first subject person is selected on the subject person list screen, the processor 111 highlights and displays the row of the subject person information of the first subject person. Note that, since the highlight display described above is for notifying of the subject person information of the selected first subject person, as long as the selected subject person can be identified, the notification may naturally be made by other methods. Next, when receiving the selection of the interview input icon 12 via the input interface 113, the processor 111 shifts to the interview input mode and outputs the interview input screen to the display via the output interface 114 (S122). Note that, here, detailed description of the interview input screen is omitted.

The processor 111 receives input of the interview information by the operator or the subject person on the interview input screen via the input interface 113 (S123). Then, the processor 111 transmits the input interview information to the server device 300 via the communication interface 115 in association with the subject person ID information of the first subject person (S124). In this way, the processor 111 ends the input process of the interview information of the first subject person.

Note that, in Figs. 8A and 8B, a case has been described in which the input of the subject person information (S114) and the input of the interview information (S123) are received as separate processes. However, after the new input of the subject person information of the subject person is performed, the interview input screen of the subject person may be displayed without change to receive the input of the interview information.

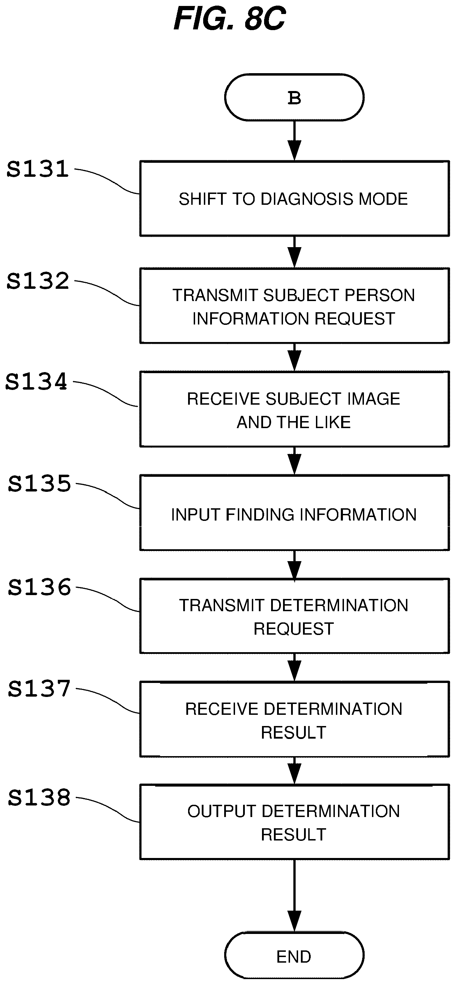

Fig. 8C is the chart illustrating the processing flow to be executed in the processing device 100 according to one embodiment of the present disclosure. Specifically, Fig. 8C is a chart illustrating a processing flow to be executed in the determination process of S41 to S45 of Fig. 7. The processing flow is mainly performed by the processor 111 of the processing device 100 reading and executing the program stored in the memory 112. Note that, although the first subject person will be described below as an example, other subject persons can be similarly processed.

According to Fig. 8C, in the subject person list screen (S112 in Fig. 8A), after receiving the selection of the desired subject person (the first subject person) to be determined via the input interface 113 (S121 in Fig. 8B), and then receiving the selection of the diagnosis icon 13, the processor 111 shifts to the diagnosis mode and outputs the diagnosis screen (S131). Further, the processor 111 transmits the subject person information request of the first subject person to the server device 300 via the communication interface 115 together with the subject person ID information of the first subject person selected in S121 of Fig. 8B (S132).

When receiving the subject person information (attribute information) of the first subject person, the subject images, the determination image information for specifying the image to be used as the determination image among the subject images from the server device 300 via the communication interface 115 (S134), the processor 111 outputs a finding input screen to the display via the output interface 114 and receives the input of the finding information (S135).

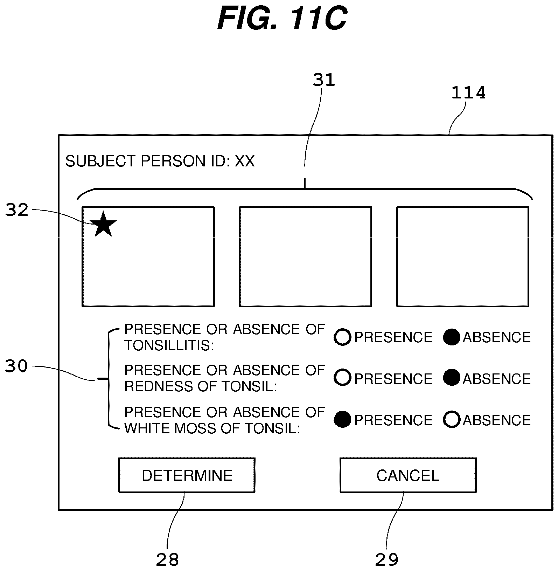

Here, Fig. 11C is the view illustrating the example of the screen to be displayed on the processing device 100 according to one embodiment of the present disclosure. Specifically, Fig. 11C is a view illustrating an example of the finding input screen of the subject person output from the processing device 100. According to Fig. 11C, the finding input screen includes an image display area 31 for displaying the transmitted subject images together with the subject person ID information of the subject person to which the findings are input. In the image display area 31, in addition to displaying the subject images captured by the imaging device 200, display 32 for specifying the image to be used as the determination image is made by being superimposed on each of the subject images. The display 32 is made based on the determination image information for specifying the image to be used as the determination image diagnosed in S134. In this way, it is possible to check which image among the subject images is the determination image. The operator such as a doctor can determine the findings for the subject person with reference to the subject images displayed in the image display area 31. Note that, although a case of displaying the subject images has been described here, as for the subject image to be displayed, all the subject images captured by the imaging device 200 may be displayed, or for example, only a part of the image specified as the determination image according to the determination image information or some of the images other than defective images in which defocus or the like has occurred may be displayed.

Further, according to Fig. 11C, the finding input screen includes a finding input area 30 for the operator such as a doctor to input the findings. In the finding input area 30, a determination item of a representative finding related to a disease to be determined and an option for selecting a determined result for the findings are output. The operator such as a doctor can input the findings by selecting a result of determining these findings via the input interface 113.

Moreover, on the finding input screen, a determination icon 28 and a cancel icon 29 are output below the finding input area 30. When selection of the determination icon 28 is received via the input interface 113, information of the findings input is transmitted to the server device 300 as the finding information, and the determination process of the possibility of contracting the predetermined disease is executed. Meanwhile, when selection of the cancel icon 29 is received, the process ends at that time, and the screen returns to the subject person list screen.

Returning to Fig. 8C again, when receiving the input of the finding information on the finding input screen in Fig. 11C, the processor 111 transmits the determination request to the server device 300 together with the finding information and the subject person ID information of the first subject person input via the communication interface 115 (S136). Then, when receiving the determination result of the possibility of contracting the predetermined disease and the determination image used for the determination from the server device 300 (S137), the processor 111 outputs the determination result and the determination image to the display via the output interface 114 (S138). In this way, the processor 111 ends the determination process for the first subject person.

### 6. Processing Flow to Be Executed by Server Device 300

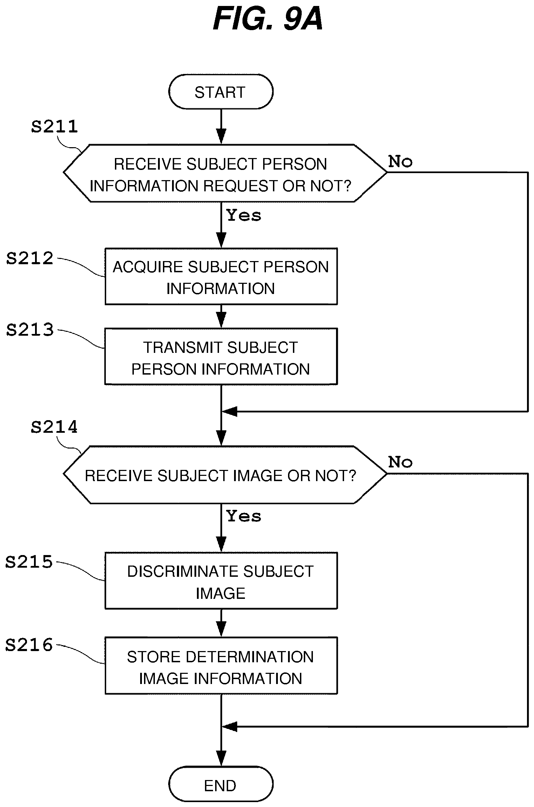

Fig. 9A is the chart illustrating the processing flow to be executed in the server device 300 according to one embodiment of the present disclosure. Specifically, Fig. 9A is a chart illustrating a processing flow to be executed in the imaging processes of S22 to S29 in the input process of the subject person information and the like of Fig. **7****.** The processing flow is mainly performed by the processor 312 of the server device 300 reading and executing the program stored in the memory 311. Note that, although the first subject person will be described below as an example, other subject persons can be similarly processed.

According to Fig. 9A, when receiving the subject person information request from the imaging device 200 via the communication interface 313 (S211), the processor 312 searches for the subject person having the status information stored as unimaged with reference to the status information in the subject person management table in the memory 311, and extracts the subject person information of the subject person (S212). Here, it is assumed that the first subject person is included as the unimaged subject person. Then, the processor 312 transmits the subject person information of the unimaged subject person including the first subject person to the imaging device 200 via the communication interface 313 (S213).

Next, the processor 312 waits until receiving the subject image from the imaging device 200 via the communication interface 313, and when receiving the subject image (S214), the processor discriminates whether or not the received subject image is the image suitable for determining the possibility of contracting the predetermined disease (S215). As an example of such discrimination, it is conceivable to perform the discrimination by inputting the received subject image to a learned determination image selection model as described below. However, the present disclosure is not limited to this method, and any other method may be used, such as a method in which the determination is made based on a coincidence with the suitable image prepared in advance by an image analysis process. Further, in a case where a plurality of images are received as the subject images, it may be discriminated whether or not the images are suitably used as the subject images. Moreover, at this time, a score as to whether or not the images are suitably used as the subject images may be calculated, and a predetermined number of images having high scores may be selected, or images having scores exceeding a predetermined threshold value may be selected.

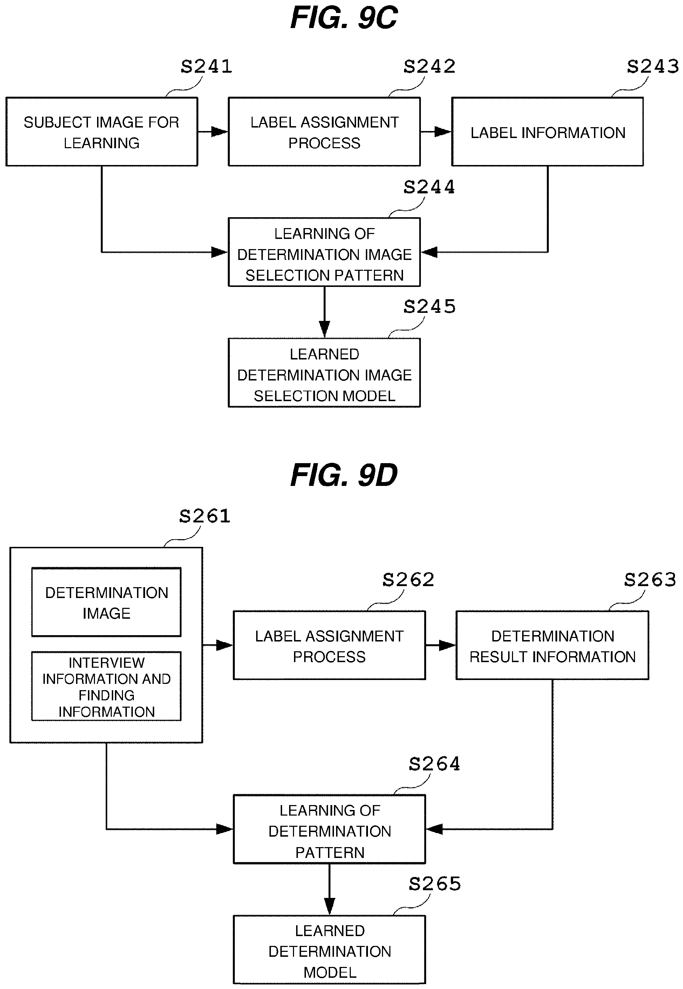

Here, Fig. 9C is the chart illustrating the processing flow related to the generation of the learned model according to one embodiment of the present disclosure. Specifically, Fig. 9C is a chart illustrating a processing flow related to generation of the learned determination image selection model used in the discrimination process of S215 in Fig. **9A****.** The processing flow may be executed by the processor 312 of the server device 300 or may be executed by a processor of another processing device.

According to Fig. 9C, the processor 312 executes a step of acquiring a subject image of a subject including at least a part of the pharynx as a subject image for learning (S241). Next, the processor 312 executes a processing step of assigning label information indicating whether or not the image can be used as the determination image to the acquired subject image for learning (S242). Then, the processor executes a step of storing the assigned label information in association with the subject image for learning (S243). Note that, in such labeling process and the storage process of the label information, the processor 312 may determine whether or not the subject image for learning is the determination image by a person in advance and store the determination image in association with the subject image for learning, or the processor 312 may analyze whether or not the subject image for learning is the determination image by a known image analysis process and store a determination result in association with the subject image for learning. Further, the label information is assigned based on viewpoints such as whether or not at least a part of the oral cavity, which is the subject, is reflected, and whether or not a quality of the image is good, free from camera shake, defocus, fogging, or the like.

When obtaining the subject image for learning and the label information associated with the subject image for learning, respectively, the processor 312 executes a step of performing machine learning of a selection pattern of the determination image using the subject image for learning and the label information (S244). As an example, the machine learning is performed by assigning a set of the subject image for learning and the label information to a neural network in combination with neurons, and repeating learning while adjusting parameters of each of the neurons so that output of the neural network becomes the same as the label information. Then, a step of acquiring the learned determination image selection model (for example, the neural network and the parameters) is executed (S245). The acquired learned determination image selection model may be stored in the memory 311 of the server device 300 or another device connected to the server device 300 via the wired or wireless network.

Returning to Fig. 9A again, the processor 312 inputs the received subject images to the learned determination image selection model generated in Fig. 9C, and discriminates whether or not each of the subject images is the image suitable for the determination of the possibility of contracting the predetermined disease (S215). Then, information for specifying the image available for the determination of the possibility of the contraction is stored in the image management table as the determination image information in association with the subject person ID information of the first subject person (S216). Further, although not particularly illustrated, the processor 312 updates the status information associated with the subject person ID information of the first subject person to "imaged" with reference to the subject person management table, and transmits the information indicating that the determination image has been obtained as the discrimination result to the processing device 100 and the imaging device 200 via the communication interface 313 together with the subject person ID information of the first subject person. Meanwhile, in a case where a predetermined number of the determination images cannot be obtained in the discrimination process, the processor 312 transmits the notification for prompting the reimaging to the imaging device 200. Such discrimination process is important for improving determination accuracy of the possibility of the contraction, but is not necessarily performed. Further, although not particularly illustrated, as illustrated in Fig. 12E, the discrimination result may be transmitted to the imaging device 200, and the operation input by the operator may be received to select whether or not to confirm the determination image. In other words, the image discriminated to be usable as the determination image may be transmitted to the imaging device 200, and previewed once, and the determination image may be finally confirmed by the server device 300 receiving that the confirmation operation has been performed. In this way, the processor 312 ends the imaging process.

Note that the number of the determination images such selected may be one or more. However, as an example, it is preferable to finally obtain from a group of about 5 to 30 subject images to a group of about 5 determination images, for example. This is because there is a high possibility to obtain better determination images by selecting the determination images from a large number of the subject images. Further, by using a plurality of determination image groups for the determination process to be described later, determination accuracy can be further improved as compared with a case where only one determination image is used. Further, as another example, every time the subject images are captured, the captured subject images may be transmitted to the processing device 100 and then the determination images may be selected, or the imaging device 200 may select the determination images, and the imaging may end in a stage in which only a predetermined number (for example, about 5) of the determination images can be acquired. In this way, it is possible to minimize time related to the imaging of the subject images while maintaining the improvement in the determination accuracy as described above. In other words, discomfort to the subject person, such as vomiting reflex can be reduced.

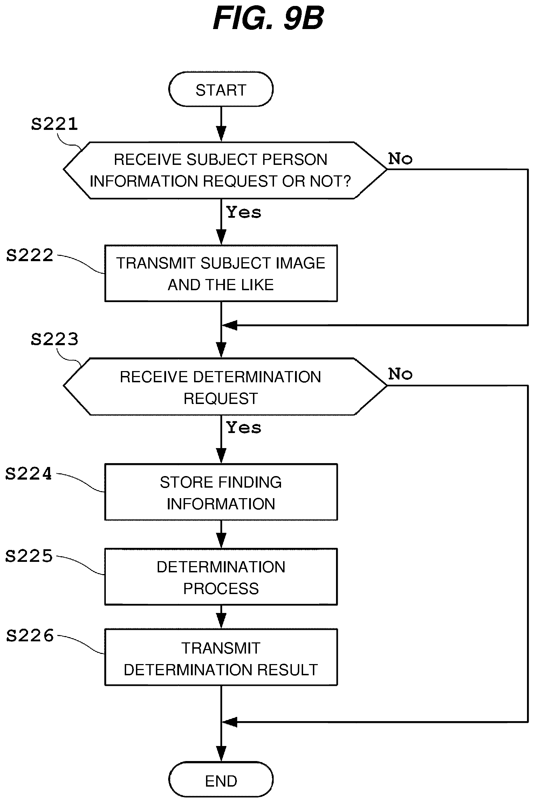

Fig. 9B is the chart illustrating the processing flow to be executed in the server device 300 according to one embodiment of the present disclosure. Specifically, Fig. 9B is a chart illustrating a processing flow to be executed in the determination processes of S42 to S44 in the input process of the subject person information and the like of Fig. 7. The processing flow is mainly performed by the processor 312 of the processing device 100 reading and executing the program stored in the memory 311. Note that, although the first subject person will be described below as an example, other subject persons can be similarly processed.

According to Fig. 9B, when receiving the subject person information request of the desired subject person (here, the first subject person) from the processing device 100 via the communication interface 313 (S221), the processor 312 acquires the attribute information of the first subject person based on the subject person ID information of the first subject person with reference to the subject person management table, and acquires the subject images associated with the subject person ID information of the first subject person and the determination image information for specifying the image to be used as the determination image among the subject images with reference to the image management table. The processor 312 transmits the acquired subject images and the like to the processing device 100 together with the attribute information of the first subject person and the subject person ID information via the communication interface 313 (S222).

Next, the processor 312 receives, from the processing device 100 via the communication interface 313, the finding information input by the operator such as a doctor and the determination request for the possibility of contracting the predetermined disease, together with the subject person ID information of the first subject person (S223). The processor 312 stores the received finding information in the subject person management table in association with the subject person ID information (S224). Next, the processor 312 reads the finding information and the interview information associated with the subject person ID information of the first subject person with reference to the subject person management table. Further, the processor 312 reads the determination image based on the determination image information associated with the subject person ID information of the first subject person with reference to the image management table. Then, the processor 312 executes the determination process based on the read information (S225). As an example of such determination process, it is conceivable to perform the determination by inputting these pieces of information to a learned determination model as described below. However, the present disclosure is not limited to this method, and any other method may be used, such as a method in which the determination is made based on a coincidence with an image indicating a contraction state by the image analysis process.

Here, Fig. 9D is the chart illustrating the processing flow related to the generation of the learned model according to one embodiment of the present disclosure. Specifically, Fig. 9D is a chart illustrating a processing flow related to generation of the learned determination model used in the determination process of S245 in Fig. 9A. The processing flow may be executed by the processor 312 of the server device 300 or may be executed by a processor of another processing device.

According to Fig. 9D, the processor 312 executes a step of acquiring the determination image selected from the subject images including at least a part of the pharynx (S261). Further, the processor 312 executes a step of acquiring the interview information and the finding information stored in advance in association with the subject person ID information of the subject person who is the subject of the determination image (S261). Next, the processor 312 executes a processing step of assigning a correct answer label assigned in advance to the subject person who is the subject of the determination image based on a result of an influenza rapid test, a PCR test, a virus isolation culture test, or the like by immunochromatography (S262). Then, the processor 312 executes a step of storing assigned correct answer label information as determination result information in association with the determination image, the interview information, and the finding information (S263). Note that, although the determination image itself is used here, a feature amount obtained from the determination image may be used.

When obtaining the determination image, the interview information, the finding information, and the correct answer label information associated therewith, respectively, the processor executes a step of performing machine learning of a determination pattern of the contraction of the disease using the determination image, the interview information, the finding information, and the correct answer label information (S264). As an example, the machine learning is performed by assigning a set of these pieces of information to the neural network in combination with the neurons, and repeating the learning while adjusting the parameters of each of the neurons so that the output from the neural network becomes the same as the correct answer label information. Then, a step of acquiring the learned determination model is executed (S265). The acquired learned determination model may be stored in the memory 311 of the server device 300 or another device connected to the server device 300 via the wired or wireless network.

Returning to Fig. 9B again, the processor 312 inputs the finding information, the interview information, and the determination image of the first subject person to the learned determination model generated in Fig. 9D, and determines the possibility of contracting the predetermined disease (S225). Then, when the determination of the possibility of the contraction is performed, the processor stores the determination result information in the subject person management table based on the subject person ID information of the first subject person, and updates the status information to "determined". Further, the processor 312 transmits the determination result information stored in the subject person management table and the determination image used for the determination to the processing device 100 together with the subject person ID information of the first subject person via the communication interface 313 (S226). In this way, the processor 312 ends the determination process.

### 7. Processing Flow to Be Executed by Imaging Device 200

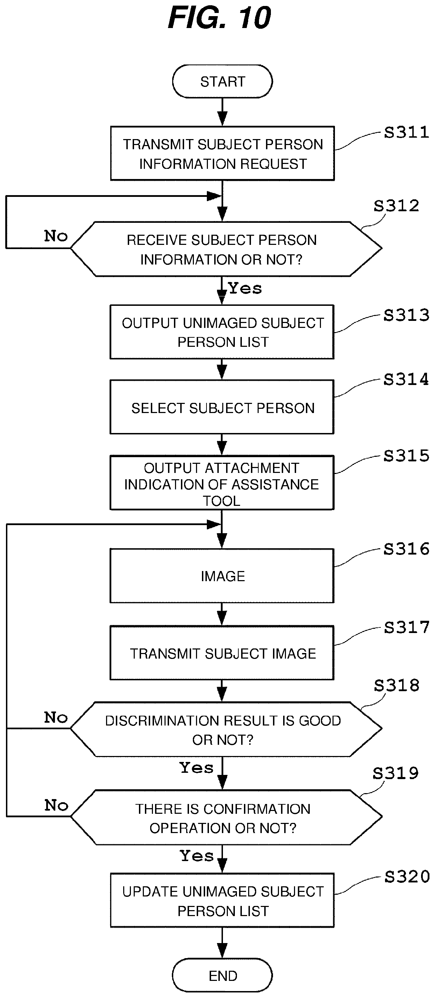

Fig. 10 is the chart illustrating the processing flow to be executed in the imaging device 200 according to one embodiment of the present disclosure. Specifically, Fig. 10 is a chart illustrating a processing flow to be executed in the imaging processes of S21 to S30 in the input process of the subject person information and the like of Fig. 7. The processing flow is mainly performed by the processor 213 of the imaging device 200 reading and executing the program stored in the memory 214. Note that, although the first subject person will be described below as an example, other subject persons can be similarly processed.

According to Fig. 10, when the imaging device 200 is started up, the processor 213 transmits the subject person information request to the server device 300 via the communication interface 216 (S311). Then, the processor 213 receives the subject person information of the unimaged subject person extracted by the server device 300 via the communication interface 216 (S312). When receiving the pieces of the subject person information of the unimaged subject persons, the processor 213 outputs the received pieces of subject person information to the display via the output interface 215 (S313).

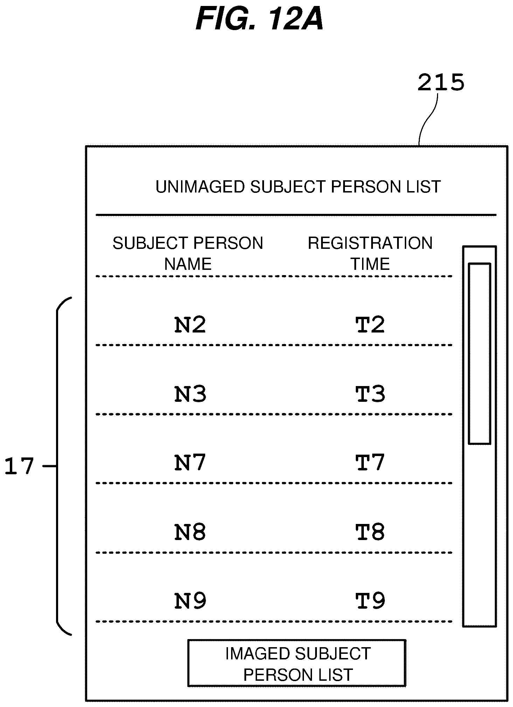

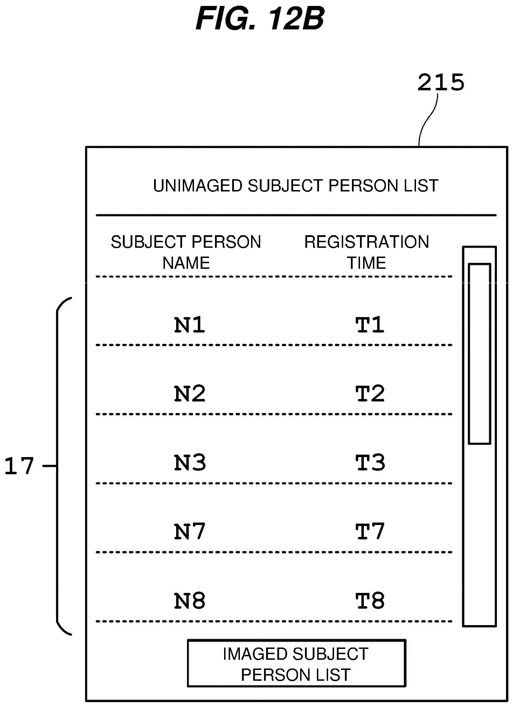

Here, Figs. 12A and 12B are views illustrating examples of a screen to be displayed on the imaging device 200 according to one embodiment of the present disclosure. Specifically, Fig. 12A illustrates an example of a screen to be displayed before the subject person information of the first subject person is newly registered and stored in the subject person management table of the server device 300. Meanwhile, Fig. 12B illustrates an example of a screen to be displayed after the subject person information of the first subject person is newly registered and stored in the subject person management table of the server device 300.

According to Fig. 12A, an unimaged subject person list screen is output on the display via the output interface 215. The unimaged subject person list screen includes a subject person list area 17 in which the pieces of the subject person information of the unimaged subject persons transmitted from the server device 300 are arranged in a list form for each row. The subject person list area 17 includes the subject person name information and time information (stored as one piece of the attribute information, although not illustrated in Fig. 6A) indicating time of the new registration for each subject person.

Here, Fig. 12A illustrates an example of a screen to be output before the subject person information of the first subject person is newly registered and stored in the subject person management table of the server device 300, as described above. Therefore, the subject person information (the subject person name information: N1, registration time information: T1) of the first subject person is not output on the unimaged subject person list screen.

Next, according to Fig. 12B, the unimaged subject person list screen similar to that in Fig. 12A is output. Here, Fig. 12B illustrates an example of a screen to be output after the subject person information of the first subject person is newly registered and stored in the subject person management table of the server device 300, as described above. Therefore, by receiving the subject person information of the first subject person in S312 of Fig. 10, the subject person information (the subject person name information: N1, the registration time information: T1) of the first subject person is output to the head (top) of the subject person list area 17 in the unimaged subject person list screen.

Note that, in Figs. 12A and 12B, each subject person information is arranged from the top in an order of most recent registration time, but the present disclosure is not limited thereto, and the subject person information can be arranged in various orders such as in an order of earliest registration time and an alphabetical order. Further, in Figs. 12A and 12B, the unimaged subject persons are displayed as a list, but for example, a screen on which the subject person information of each of the unimaged subject persons (or each of a predetermined number of the unimaged subject persons) is displayed may be sequentially switched and displayed, and selection may be received when a desired subject person is displayed.

Returning to Fig. 10 again, the processor 213 receives selection of a desired subject person to be imaged via the input interface 210 on the unimaged subject person list screen displayed in S313 (S314). As an example, on the screens of Fig. 12A or 12B, a tap operation is made on subject person name information of the desired subject person to be imaged on the unimaged subject person list screen, whereby the subject person to be imaged is selected. When the subject person is selected, the processor 213 outputs attribute information of the selected subject person to the display via the output interface 215.

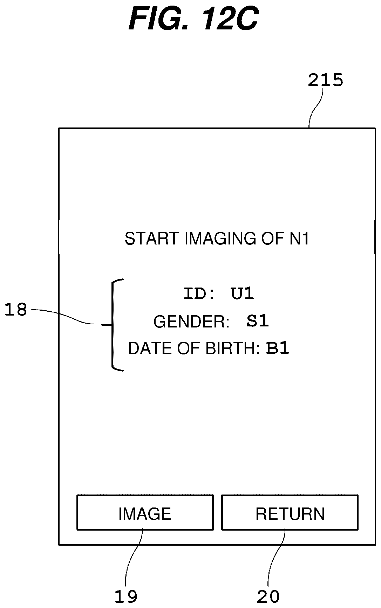

Here, Fig. 12C is the view illustrating the example of the screen to be displayed on the imaging device 200 according to one embodiment of the present disclosure. Specifically, Fig. 12C is a view illustrating an example of an attribute information display screen to be displayed in a case where the first subject person is selected in S314 of Fig. 10. According to Fig. 12C, the attribute information display screen includes an attribute display area 18. In the attribute display area 18, in addition to the subject person ID information of the first subject person, the gender information and the date of birth information are displayed as the attribute information of the first subject person received from the server device 300. In this way, the operator can check detailed information of the first subject person, and it is possible to reduce the possibility of the mistaking of the subject person by the subject person himself/herself performing identity confirmation based on the information displayed here. In other words, it is possible to reliably associate the subject image of the subject person to be imaged later with the subject person ID information of the first subject person.

Further, according to Fig. 12C, an "imaging icon 19" and a "return icon 20" are output below the attribute display area 18. When selection of the "imaging icon 19" is received via the input interface 210, the camera 211 is started up to image the subject. Meanwhile, when selection of the "return icon 20" is received via the input interface 210, the screen returns to the unimaged subject person list screen again.

Returning to Fig. 10 again, the processor 213 determines whether or not the assistance tool 400 is normally attached to the imaging device 200, and outputs the attachment indication for prompting the attachment of the assistance tool 400 in a case where it is determined that the assistance tool is not attached (S315). Note that such determination may be performed by any method, and examples of the method include a method of detecting that a predetermined switch is turned on by the attachment of the assistance tool 400, a method of detecting presence or absence of a pattern specific to an image captured in a state where the assistance tool 400 is attached, or a combination thereof. Further, in S315, the determination of the attachment of the assistance tool 400 is made, but this process itself may be skipped. Further, instead of the determination of the attachment of the assistance tool 400, it may also be possible for the operator himself/herself to input that the assistance tool 400 has been attached by, for example, making it possible to output the check indication for the operator to check the attachment of the assistance tool 400 via the output interface 215, and receive the predetermined operation input (for example, the tap operation) for the check indication by the operator.

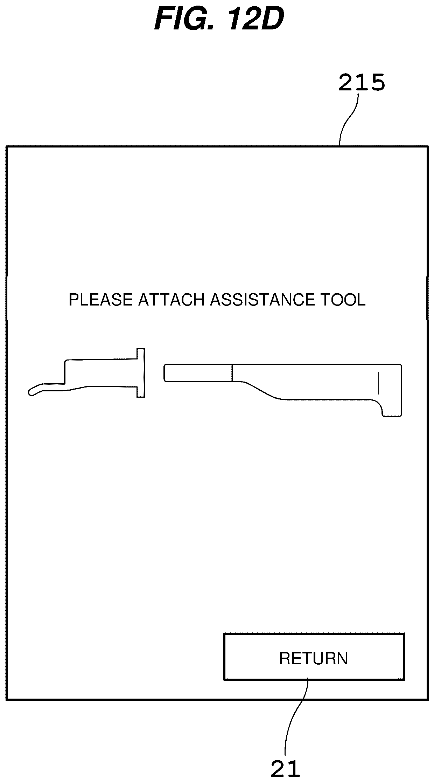

Here, Fig. 12D is the view illustrating the example of the screen to be displayed on the imaging device 200 according to one embodiment of the present disclosure. Specifically, Fig. 12D is a view illustrating an example of an attachment indication screen to be displayed in S315 of Fig. 10. According to Fig. 12D, the attachment indication screen includes the indication of "please attach the assistance tool" for promoting the attachment of the assistance tool 400, and images imitating the assistance tool 400 and the imaging device 200. As an example, the screen transitions so that both the images imitating the assistance tool 400 and the imaging device 200 gradually approach each other over time, and it is possible to more intuitively promote the operator to attach the assistance tool 400. Note that, although the "return icon 21" is output on the screen, the screen can return to the attribute information display screen by receiving the selection of the icon.

Returning to Fig. 10 again, as a result of determining whether or not the assistance tool 400 is attached at a predetermined cycle in a state where the attachment indication screen of the assistance tool is output, if it is determined that the assistance tool is normally attached, the processor 213 starts the imaging the inner side of the oral cavity including the pharynx as the subject by the camera 211 (S316). Specifically, the imaging is performed by turning on the light source 212, irradiating the subject with the light, and detecting the reflected light from the subject such as the pharynx by the camera 211.

Here, Fig. 12E is the view illustrating the example of the screen to be displayed on the imaging device 200 according to one embodiment of the present disclosure. Specifically, Fig. 12E is a view illustrating an example of an imaging screen to be output in S316 of Fig. 10. According to Fig. 12E, the imaging screen includes a through image area 32, and a current subject image detected by the camera 211 is output to the through image area 32 (a specific image is not illustrated). Further, at this time, in the through image area 32, a guide 22 drawn along a general shape of the pharynx, which is the subject, is superimposed on the through image and output. By outputting such guide 22 superimposed on the through image, a position in a left-right direction and a size in the depth direction of the subject being currently captured can be adjusted with reference to the guide 22.

Further, according to Fig. 12E, an imaging icon 23 and a return icon 24 are output below the through image area 32. By receiving an operation input to the imaging icon 23 via the input interface 210, fetching of the image captured by the camera 211 as the subject image as the still image or the moving image is started. Note that the operation input to the imaging icon 23 is merely an example of imaging start, and may be started by another method such as detection of pressing down the capture button, for example. Further, it is possible to return to the attribute display screen by receiving an operation input to the return icon 24 via the input interface 210.

Returning to Fig. 10 again, when the one or the plurality of subject images are acquired by performing the imaging as described above, the processor 213 transmits the subject image captured via the communication interface 216 to the server device 300 together with the subject person ID information of the first subject person (S317). The processor 213 waits until receiving a discrimination result of the subject image from the server device 300, and when receiving the information indicating that the image suitable for the determination of the possibility of the contraction can be acquired, outputs the result to the display via the output interface 215 (S318).

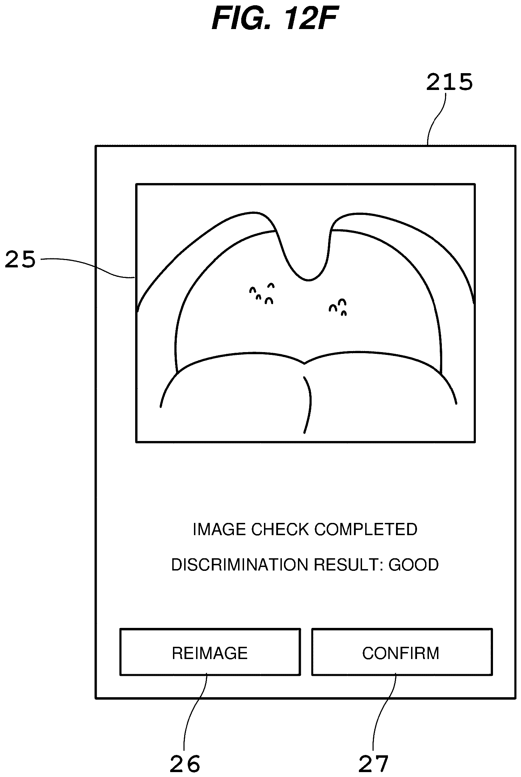

Here, Fig. 12F is the view illustrating the example of the screen to be displayed on the imaging device 200 according to one embodiment of the present disclosure. Specifically, Fig. 12F is a view illustrating an example of a discrimination result screen to be output in S318 of Fig. 10. According to Fig. 12F, the discrimination result screen includes a preview area 25 of the determination image. In the preview area 25, the subject image selected as the determination image is output to the operator as a result of the discrimination in the server device 300. The operator can check the determination image output to the preview area 25, and select whether to perform the imaging again or confirm the determination image.

Further, information indicating a discrimination result by the server device 300 is displayed below the preview area 25. Here, display of "good" indicating that the determination image suitable for determining the possibility of contracting the predetermined disease has been obtained is made. In this way, the operator can confirm that the subject image available for the determination can be captured.

Moreover, a reimaging icon 26 and a confirmation icon 27 are output below the information indicating the discrimination result. When receiving selection of the reimaging icon 26 via the input interface 210, the processor 213 transmits information indicating the reimaging to the server device 300, starts up the camera 211, and returns to the imaging process in S316 again. Meanwhile, when receiving selection of the confirmation icon 27 via the input interface 210, the processor 213 transmits information indicating that a determination image confirmation operation has been performed to the server device 300.

In other words, by outputting the discrimination result as illustrated in Fig. 12F as to whether or not the image suitable for the determination of the possibility of the contraction can be acquired in S318 of Fig. 10, it is possible to notify the operator of the necessity of the reimaging immediately after the imaging of the subject image (after the processing of S316), and the imaging can be performed more smoothly. As an example, the determination process of the possibility of contracting the predetermined disease usually takes a large processing load and a long time. Therefore, the process of discriminating whether or not the image is a suitable image that can be processed in a short time with a lighter processing load is first executed, and a result of the determination is output, whereby the time until the reimaging can be reduced. Further, even if it has been found that the reimaging is necessary immediately before the determination of the possibility of contracting the predetermined disease, the assistance tool 400 and the like have already been removed and discarded, and it is necessary to attach a new assistance tool 400 again. As described above, such inconvenience can be eliminated by notifying at a timing immediately after the subject image is captured.

Note that Fig. 12F illustrates a case where the discrimination result and the preview area 25 of the determination image are displayed together on the discrimination result screen. However, the present disclosure is not limited thereto, and the discrimination result and the preview area 25 of the determination image may be displayed on separate screens, and may be switched and displayed by a switching operation of the operator.

Returning to Fig. 10 again, when receiving selection of the confirmation icon 27 illustrated in Fig. 12F via the input interface 210, the processor 213 transmits information indicating that the confirmation operation has been made to the server device 300 via the communication interface 216. Then, the processor 213 again receives the subject person information of the unimaged subject person via the communication interface 216, updates and outputs the subject person information to the unimaged subject person list screen of the display via the output interface 215 (S320). At this time, since the status information of the first subject person is "imaged", the status information is not included in the subject person information of the above unimaged subject person. Therefore, the unimaged subject person list screen does not include the subject person information of the first subject person. Note that it is possible to output a list of imaged subject persons by selecting an imaged list icon on the unimaged subject person list screen, and the subject person information of the first subject person is output to the list.

As described above, in the present embodiment, it is possible to provide the imaging device, the program, and the method more suitable for capturing the image of the subject including at least the part of the oral cavity of the subject person.

### 8. Variations

In the above embodiment, a case of outputting the information indicating the possibility of contracting the influenza using the subject image, the interview information, and the finding information has been described. However, instead of or in addition to the interview information and the finding information, the information indicating the possibility of contracting the influenza may be output using external factor information related to the influenza. Examples of such external factor information include a determination result made for another subject, a diagnosis result by a doctor, and influenza epidemic information in an area to which the subject person belongs. The processor 312 acquires such external factor information from another processing device or the like via the communication interface 313, and assigns the external factor information as an input to the learned determination model, whereby a positive rate considering the external factor information can be obtained.

Further, in the above embodiment, a case of determining the possibility of contracting the influenza using the interview information and the finding information in addition to the subject image has been described. However, the present disclosure is not limited thereto, and the determination can also be made using only the subject image.

In the above embodiment, a case has been described in which the interview information and the finding information are input in advance by the operator or the subject person, or received from the electronic medical record device or the like connected to the wired or wireless network. However, these pieces of information may also be obtained from the subject image captured instead of or in addition to these. The finding information and the interview information associated with the subject image for learning are assigned as the correct answer labels to the subject image for learning, and these sets are subjected to the machine learning via the neural network to obtain a learned information estimation model. Then, the processor 111 assigns the subject image to the learned information estimation model as an input, whereby desired interview information and attribute information can be obtained. Examples of such interview information and attribute information include a gender, an age, a degree of pharyngeal redness, a degree of tonsillar swelling, and presence or absence of white moss. In this way, it is possible to save time and effort for the operator to input the interview information and the finding information.

Each learned model described in the above embodiment is generated using the neural network or a convolutional neural network. However, the present disclosure is not limited thereto, and the generation can also be performed using machine learning such as a nearest-neighbor method, a decision tree, a regression tree, and a random forest.

In the above embodiment, a case has been described in which the discrimination process of the determination image and the determination process are performed in the server device 300. However, these various processes can be appropriately distributed and performed by the processing device 100, the imaging device 200, or other devices (including a cloud server device and the like).

Note that these variations are similar to the configurations, the processes, and the procedures in one embodiment described with reference to Figs. 1 to 12F, except for points specifically described above. Accordingly, detailed description of these matters will be omitted. Further, it is also possible to configure the system by appropriately combining or replacing each of the elements described in each of the variations or each of the embodiments.

The processes and the procedures described in the present description can also be implemented not only by those explicitly described in the embodiments but also by software, hardware, or a combination thereof. Specifically, the processes and the procedures described in the present description are implemented by installing logic corresponding to the processes on a medium such as an integrated circuit, a volatile memory, a nonvolatile memory, a magnetic disk, or an optical storage. Further, the processes and the procedures described in the present description can be implemented as a computer program and executed by various computers including the processing device and the server device.

Even if it is described that the processes and the procedures described in the present description are executed by a single device, software, a component, or a module, such processes or procedures can be executed by a plurality of devices, a plurality of pieces of software, a plurality of components, and/or a plurality of modules. Further, even if it is described that various types of information described in the present description are stored in a single memory or storage unit, such information can be stored in a distributed manner in a plurality of memories included in a single device or the plurality of memories arranged in a distributed manner in a plurality of devices. Moreover, software and hardware elements described in the present description can be achieved by integrating the elements into fewer components or decomposing the elements into more components.

### Reference Signs List

- 1: Processing system
- 100: Processing device
- 200: Imaging device
- 300: Server device
- 400: Assistance tool
- 600: Operator
- 700: Subject person

## Claims

1. An imaging device comprising: a camera configured to capture an image of a subject including at least a part of a natural opening of one or a plurality of subject persons including a first subject person; and at least one processor, wherein
the at least one processor is configured to perform a process of
receiving subject person information including first subject person information associated with the one or each of the plurality of subject persons including the first subject person via a communication interface from an external device communicably connected to the imaging device via a network, and
in a case of outputting, as a list, pieces of subject person information of unimaged subject persons among the one or the plurality of subject persons, outputting the list without including the first subject person information before the first subject person information associated with the first subject person is registered in the external device, and outputting the list including the first subject person information after the first subject person information is registered in the external device.

2. The imaging device according to claim 1, wherein the at least one processor is configured to perform a process of outputting attribute information of the first subject person when receiving selection of the first subject person information from the list output including the first subject person information.

3. The imaging device according to claim 1 or 2, wherein the at least one processor is configured to, after receiving selection of the first subject person information from the list output including the first subject person information, perform a process of transmitting the first subject person information and a first subject image to the external device in association with each other when the first subject image including at least a part of a natural opening of the first subject person is captured by the camera.

4. The imaging device according to claim 3, wherein the first subject image is used to determine a possibility of contracting a predetermined disease in the external device.

5. The imaging device according to claim 4, wherein the at least one processor is configured to perform a process of receiving discrimination information indicating whether or not the first subject image is suitable for use in the determination from the external device, and outputting the received discrimination information.

6. The imaging device according to any one of claims 1 to 5, wherein the at least one processor is configured to perform a process of outputting an attachment indication that prompts attachment of an assistance tool to be inserted into the natural opening together with at least a part of the imaging device by covering the part.

7. A program that causes an imaging device including a camera configured to capture an image of a subject including at least a part of a natural opening of one or a plurality of subject persons including a first subject person to function as a processor, wherein
the processor is to
receive subject person information including first subject person information associated with the one or each of the plurality of subject persons including the first subject person via a communication interface from an external device communicably connected to the imaging device via a network, and
in a case of outputting, as a list, pieces of subject person information of unimaged subject persons among the one or the plurality of subject persons, output the list without including the first subject person information before the first subject person information associated with the first subject person is registered in the external device, and output the list including the first subject person information after the first subject person information is registered in the external device.

8. A method to be executed by at least one processor in an imaging device including: a camera configured to capture an image of a subject including at least a part of a natural opening of one or a plurality of subject persons including a first subject person; and the at least one processor, wherein
the method includes:
a stage of receiving subject person information including first subject person information associated with the one or each of the plurality of subject persons including the first subject person via a communication interface from an external device communicably connected to the imaging device via a network, and
a stage of, in a case of outputting, as a list, pieces of subject person information of unimaged subject persons among the one or the plurality of subject persons, outputting the list without including the first subject person information before the first subject person information associated with the first subject person is registered in the external device, and outputting the list including the first subject person information after the first subject person information is registered in the external device.
